# EUROPÄISCHE PATENTANMELDUNG

(11) **EP 2 098 226 A1**
(43) Veröffentlichungstag der Anmeldung: **09.09.2009**
(21) Anmeldenummer: 08004187.4
(22) Anmeldetag: 06.03.2008
(51) Int. Cl.: A61K 31/045, A61K 31/05, A61K 31/06, A61K 31/12, A61K 31/136, A61K 31/167, A61K 31/194, A61P 9/04

(54) **AKAP-PKA-Interaktionshemmer zur Anwendung in der Behandlung von Herzkrankheiten**

(71) Anmelder: Forschungsverbund Berlin e.V., 12489 Berlin (DE)
(72) Erfinder: Klussmann, Enno, Dr., 14163 Berlin (DE); Rosenthal, Walter, 14532 Kleinmachnow (DE); Frank, Christian, 12049 Berlin (DE); Genieser, Hans-Gottfried, 27809 Lemwerder (DE); Zimmermann, Bastian, 34121 Kassel (DE); Szaszak, Marta, Dr., 10439 Berlin (DE)
(74) Vertreter: Forstmeyer, Dietmar

(57) **Zusammenfassung**

Die Erfindung betrifft neue Verbindungen der Formeln (X) und (I), 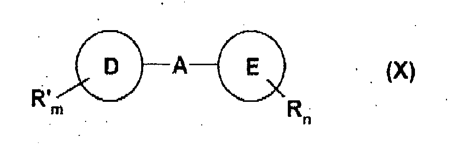 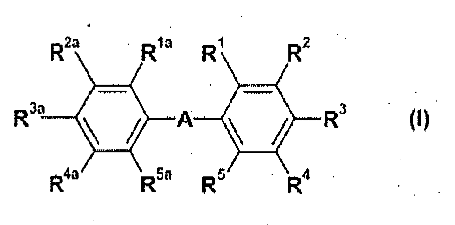 die die Interaktion von Proteinkinase A (PKA) und Proteinkinase A-Ankerproteinen (AKAP) modulieren, insbesondere inhibieren. Die Erfindung betrifft weiterhin pharmazeutische Mittel, insbesondere für die Behandlung von Krankheiten, die mit einer Störung des cAMP Signalweges assoziiert sind, insbesondere Krankheiten wie Hypertrophie des Herzens, koronare Herzkrankheiten, Hypertonie und Herzinsuffizienz.

## Beschreibung

Die Erfindung betrifft neue Verbindungen, die die Interaktion von Proteinkinase A (PKA) und Proteinkinase A-Ankerproteinen (AKAP) modulieren, insbesondere inhibieren. Die Erfindung betrifft weiterhin pharmazeutische Mittel, insbesondere für die Behandlung von Krankheiten, die mit einer Störung des cAMP Signalweges assoziiert sind, insbesondere Krankheiten wie Hypertrophie des Herzens, koronare Herzkrankheiten, Hypertonie und Herzinsuffizienz.

AKAP (Proteinkinase A Ankerproteine) sind Gerüstproteine, die direkt mit der PKA (Proteinkinase A) interagieren. Sie verankern die PKA an zellulären Kompartimenten und koordinieren dadurch kompartimentalisierte cAMP/PKA-abhängige Signalverarbeitung (cAMP = Cyclisches Adenosinmonophosphat). AKAP-PKA-Interaktionen spielen bei der Kontraktion von Kardiomyozyten und bei der AVP-vermittelten Wasserrückresorption (AVP = Arginin-Vasopressin) in renalen Hauptzellen eine wichtige Rolle. Beide Zelltypen sind z.B. bei Herzinsuffizienz funktionell entscheidend.

Herzinsuffizienz ist heute weltweit die Herzkreislauferkrankung mit der schnellsten Zuwachsrate und die häufigste Ursache für Krankenhauseinweisungen älterer Personen. An Herzinsuffizienz leiden weltweit ca. 22. Millionen Menschen und die Behandlungskosten betragen ca. USD 80 Mrd. (Business Insights. The Cardiovascular Outlook to 2008).

Bisher wurden zur Therapie der Herzinsuffizienz positiv inotrope Substanzen und klassische Antihypertensiva wie Diuretika, beta-Blocker, ACE-Hemmer und AT1-Rezeptorblocker eingesetzt.

Die gegenwärtige Pharmakotherapie, insbesondere für späte Stadien der Herzinsuffizienz, ist mit unerwünschten Nebenwirkungen, z. B. einer um 28 % erhöhten Mortalität unter Behandlung mit dem PDE3-Hemmer Milrinon (positiv inotrope Substanz) oder Störungen der Na+- und K+-Spiegel im Blutplasma bei Behandlung mit Diuretika behaftet.

Für Herzinsuffizienz, insbesondere für späte Stadien der Erkrankungen, steht keine ausreichende Therapie zur Verfügung.

Die Aufgabe der vorliegenden Erfindung bestand daher darin, neue Verbindungen bereitzustellen, die die AKAP-PKA Interaktion hemmen. Insbesondere bestand die Aufgabe darin, neue Verbindungen bereitzustellen, die zur Behandlung von Herzinsuffizienz verwendet werden können.

Diese Aufgabe wird durch die Verbindungen der vorliegenden Erfindung gelöst, die AKAP-PKA-Interaktionen durch Bindung an regulatorische Untereinheiten der PKA hemmen und gleichzeitig PKA aktivieren. Die Substanzen können z.B. zur Therapie von Herzinsuffizienz eingesetzt werden. Der molekulare Wirkmechanismus und Effekte der Substanzen auf Zellen und isolierte Herzen sind in den beiliegenden Abbildungen dargestellt.

Es handelt sich hierbei um eine neue Strategie, nämlich die pharmakologische Intervention an klinisch relevanten Protein-Protein-Wechselwirkungen in definierten Kompartimenten von Zellen. Durch die Spezifität von Protein-Protein-Interaktionen ist ein gezielterer pharmakologischer Eingriff in zelluläre Prozesse möglich als durch konventionelle Therapien, die in der Regel auf die Modulation von Enzymen, Rezeptoren und Ionenkanälen bzw. -pumpen abzielen und dadurch Funktionen von Proteinen in der gesamten Zelle beeinflussen und unerwünschte Nebenwirkungen hervorrufen können.

Die vorliegende Erfindung betrifft Verbindungen der Formel (X), 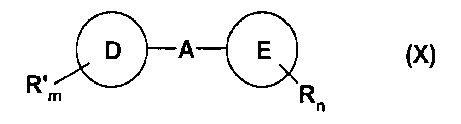 wobei
A ein Sauerstoffatom, ein Schwefelatom, eine NH, CO, Alkylen, Alkenylen, Alkinylen, Heteroalkylen, Arylen, Heteroarylen, Cycloalkylen, Alkylcycloalkylen, Heteroalkylcycloalkylen, Heterocycloalkylen, Aralkylen oder eine Heteroaralkylengruppe ist,
die Reste R unabhängig voneinander ein Wasserstoffatom, ein Halogenatom, ein NH₂, OH, SH, N₃, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
die Reste R' unabhängig voneinander ein Wasserstoffatom, ein Halogenatom, ein NH₂, OH, SH, N₃, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
D ein Aryl-, Heteroaryl-, Cycloalkyl-, Alkylcycloalkyl-, Heteroalkylcycloalkyl-, Heterocycloalkyl-, Aralkyl- oder ein Heteroaralkylring ist,
E ein Aryl-, Heteroaryl-, Cycloalkyl-, Alkylcycloalkyl-, Heteroalkylcycloalkyl-, Heterocycloalkyl-, Aralkyl- oder ein Heteroaralkylring ist,
n gleich 0, 1, 2, 3, 4 oder 5 ist und
m gleich 0, 1, 2, 3, 4 oder 5 ist,
oder ein pharmakologisch akzeptables Salz, Solvat, Hydrat oder eine pharmakologisch akzeptable Formulierung derselben.

Bevorzugt sind Verbindungen der Formel (X), wobei A ein Sauerstoffatom, ein Schwefelatom, eine NH, CO, Alkylen, Alkenylen, oder Heteroalkylengruppe ist.

Weiter bevorzugt sind Verbindungen der Formel (X), wobei D eine Phenylgruppe oder eine Heteroarylgruppe mit 5 oder 6 Ringatomen ist.

Weiter bevorzugt sind Verbindungen der Formel (X), wobei E eine Phenylgruppe oder eine Heteroarylgruppe mit 5 oder 6 Ringatomen ist.

Weiter bevorzugt sind Verbindungen der Formel (X), wobei die Reste R unabhängig voneinander ein ein Wasserstoffatom, ein Halogenatom, eine NH₂, OH, NO₂, Alkyl, Alkenyl, Alkinyl oder Heteroalkylgruppe sind.

Weiter bevorzugt sind Verbindungen der Formel (X), wobei die Reste R' unabhängig voneinander ein Wasserstoffatom, ein Halogenatom, eine NH₂, OH, NO₂, Alkyl, Alkenyl, Alkinyl oder Heteroalkylgruppe sind.

### Weiter bevorzugt ist n 1, 2 oder 3 (insbesondere 1 oder 2)

Weiter bevorzugt ist m 1, 2 oder 3 (insbesondere 1 oder 2)

Die vorliegende Erfindung betrifft ferner Verbindungen der Formel (I), 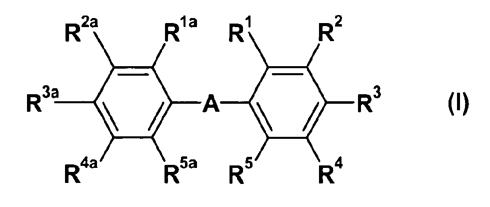 wobei
A ein Sauerstoffatom, ein Schwefelatom, eine NH, CO, Alkylen, Alkenylen, Alkinylen, Heteroalkylen, Arylen, Heteroarylen, Cycloalkylen, Alkylcycloalkylen, Heteroalkylcycloalkylen, Heterocycloalkylen, Aralkylen oder eine Heteroaralkylengruppe ist und
die Reste R¹, R^{1a}, R², R^{2a}, R³, R^{3a}, R⁴, R^{4a}, R⁵ und R^{5a} unabhängig voneinander ein Wasserstoffatom, ein Halogenatom, ein NH₂, OH, N₃, SH, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
oder ein pharmakologisch akzeptables Salz, Solvat, Hydrat oder eine pharmakologisch akzeptable Formulierung derselben.

Die vorliegende Erfindung betrifft insbesondere Verbindungen der Formel (I), 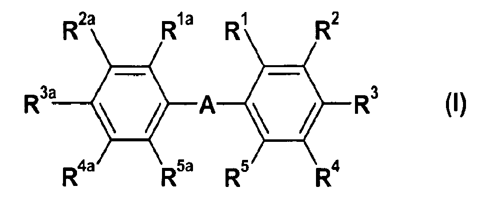 wobei
A ein Sauerstoffatom, ein Schwefelatom, eine NH, CO, Alkylen, Alkenylen, oder Heteroalkylengruppe ist,
R¹ ein Wasserstoffatom, ein Halogenatom, eine NH₂, OH, NO₂, Alkyl, Alkenyl, Alkinyl oder Heteroalkylgruppe ist,
R^{1a} ein Wasserstoffatom, ein Halogenatom, eine NH₂, OH, NO₂, Alkyl, Alkenyl, Alkinyl oder Heteroalkylgruppe ist,
R² ein Wasserstoffatom, ein Halogenatom, eine NH₂, OH, NO₂, Alkyl, Alkenyl, Alkinyl oder Heteroalkylgruppe ist,
R^{2a} ein Wasserstoffatom, ein Halogenatom, eine NH₂, OH, NO₂, Alkyl, Alkenyl, Alkinyl oder Heteroalkylgruppe ist,
R³ ein Wasserstoffatom, ein Halogenatom, eine NH₂, OH, NO₂, Alkyl, Alkenyl, Alkinyl oder Heteroalkylgruppe ist,
R^{3a} ein Wasserstoffatom, ein Halogenatom, eine NH₂, OH, NO₂, Alkyl, Alkenyl, Alkinyl oder Heteroalkylgruppe ist,
R⁴ ein Wasserstoffatom, ein Halogenatom, eine NH₂, OH, NO₂, Alkyl, Alkenyl, Alkinyl oder Heteroalkylgruppe ist,
R^{4a} ein Wasserstoffatom, ein Halogenatom, eine NH₂, OH, NO₂, Alkyl, Alkenyl, Alkinyl oder Heteroalkylgruppe ist,
R⁵ ein Wasserstoffatom, ein Halogenatom, eine NH₂, OH, NO₂, Alkyl, Alkenyl, Alkinyl oder Heteroalkylgruppe ist,
R^{5a} ein Wasserstoffatom, ein Halogenatom, eine NH₂, OH, NO₂, Alkyl, Alkenyl, Alkinyl oder Heteroalkylgruppe ist, oder
die Reste R¹ und R^{1a} oder die Reste R⁵ und R^{5a} zusammen eine Bindung sind,
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 12 Kohlenstoffatome, vorzugsweise 1 bis 6 Kohlenstoffatome, besonders bevorzugt 1 bis 4 Kohlenstoffatome aufweist, z.B. die Methyl-, Ethyl-, Propyl-, Isopropyl-, n-Butyl-, Isobutyl-, tert-Butyl, n-Pentyl-, n-Hexyl-, 2,2-Dimethylbutyl- oder n-Octyl-Gruppe.

Der Ausdruck Alkylen bezieht sich auf eine Alkylgruppe, die an zwei Reste gebunden ist, z. B. eine -CH₂-, -CH₂CH₂-, -CH(CH₃)-, -C(CH₃)₂-, -CH(CF₃)- oder eine -C(CF₃)₂- Gruppe.

Die Ausdrücke Alkenyl und Alkinyl beziehen sich auf zumindest teilweise ungesättigte, geradkettige oder verzweigte Kohlenwasserstoffgruppen, die 2 bis 12 Kohlenstoffatome, vorzugsweise 2 bis 6 Kohlenstoffatome, besonders bevorzugt 2 bis 4 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Der Ausdruck Alkenylen bezieht sich auf eine Alkenylgruppe, die an zwei Reste gebunden sind, wie z.B. eine -CH=CH- oder eine -C(CH₃)=CH- Gruppe. Ebenso bezieht sich die Endung "en" auf die entsprechende Gruppe, die an zwei Reste gebunden ist (z.B. die Ringe D und E).

Des Weiteren beziehen sich die Begriffe Alkyl, Alkylen, Alkenyl, Alkenylen und Alkinyl auf Gruppen, bei der ein oder mehrere Wasserstoffatome unabhängig voneinander durch ein Halogenatom (bevorzugt F oder Cl) ersetzt sind wie z. B. die 2,2,2-Trichlorethyl-, oder die Trifluormethylgruppe.

Der Ausdruck Heteroalkyl (bzw. Heteroalkylen) bezieht sich auf eine Alkyl-, eine Alkylen-, eine Alkenyl-, eine Alkenylen- oder eine Alkinyl-Gruppe (z. B. Heteroalkenyl, Heteroalkenylen, Heteroalkinyl), in der ein oder mehrere (bevorzugt 1, 2 oder 3) Kohlenstoffatome unabhängig voneinander durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe wie z. B. Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R³-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CQ-N(R^{b})-Y^{a}-, R^{a}-N(R^{b})-CO-S-Y^{a}-, R^{a}- S-CO-O-Y^{a}-, R^{a}-O-CO-S-Y^{a}-, R^{a}-S-CQ-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-,
wobei R^{a} ein Wasserstoffatom, eine C₁-C₆-Alkyl-, eine C₂-C₆-Alkenyl- oder eine C₂-C₆-Alkinylgruppe; R^{b} ein Wasserstoffatom, eine C₁-C₆-Alkyl-, eine C₂-C₆-Alkenyl- oder eine C₂-C₆-Alkinylgruppe; R^{c} ein Wasserstoffatom, eine C₁-C₆-Alkyl-, eine C₂-C₆-Alkenyl- oder eine C₂-C₆-Alkinylgruppe; R^{d} ein Wasserstoffatom, eine C₁-C₆-Alkyl-, eine C₂-C₆-Alkenyl- oder eine C₂-C₆-Alkinylgruppe und Y^{a} eine direkte Bindung, eine C₁-C₆-Alkylen-, eine C₂-C₆-Alkenylen- oder eine C₂-C₆-Alkinylengruppe ist,
wobei jede Heteroalkylgruppe mindestens ein Kohlenstoffatom enthält und ein oder mehrere Wasserstoffatome durch Fluor- oder Chloratome ersetzt sein können.

Bevorzugt sind Heteroalkylgruppen Gruppen der Formeln R^{a}-O-_{Y}^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}- und R^{a}-N(R^{b})-CO-Y^{a}-, wobei R^{a} ein Wasserstoffatom oder eine C₁-C₆-Alkylgruppe ist; R^{b} ein Wasserstoffatom oder eine C₁-C₆-Alkylgruppe ist und Y^{a} eine Bindung oder eine C₁-C₆-Alkylen-, gruppe ist.

Konkrete Beispiele für Heteroalkylgruppen sind Methoxy, Trifluormethoxy, Ethoxy, CH₂OH, NHCOCH₃, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methyl-aminomethyl, Ethylaminomethyl, Di-iso-Propylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitril-gruppen. Beispiele für eine Heteroalkylengruppe sind Gruppen der Formeln -CH₂CH(OH)-, -CH(OH)-, -CO-, -CH₂CO- und -COCH₂-.

Der Ausdruck Halogenatom bezieht sich insbesondere auf ein F, Cl, Br oder I Atom, vorzugsweise auf F oder Cl, insbesondere Cl.

Der Ausdruck Cycloalkyl bezieht sich auf eine gesättigte oder teilweise ungesättigte (z.B. Cycloalkenyl) cyclische Gruppe, die einen oder mehrere Ringe (bevorzugt 1 oder 2) aufweist, und 3 bis 14 Ringkohlenstoffatome, vorzugsweise 3 bis 10 (insbesondere 3, 4, 5, 6 oder 7) Ringkohlenstoffatome enthält. Der Ausdruck Cycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z.B. cyclische Ketone wie z.B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]decanyl-, Norbornyl-, Cyclohexyl-, Cyclopentenyl-, Cyclohexadienyl-, Decalinyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ringkohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind. Beispiele sind die Piperidyl-, Piperazinyl-, Morpholinyl-, Urotropinyl-, Pyrrolidinyl-, Tetrahydrothiophenyl-, Tetrahydropyranyl-, Tetrahydrofuryl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten, z.B. Alkylcycloalkyl-, Cycloalkylalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringe aufweist und 3 bis 10 (insbesondere 3, 4, 5, 6 oder 7) Kohlenstoffatome enthält und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringsysteme mit 3 bis 10 (insbesondere 3, 4, 5, 6 oder 7) Ringatomen und eine oder zwei Alkyl-, Alkenyl-, Alkinyl- oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe aufweist, und 6 bis 14 Ringkohlenstoffatome, vorzugsweise 6 bis 10 (insbesondere 6) Ringkohlenstoffatome enthält. Der Ausdruck Aryl (bzw. Ar) bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl-, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe aufweist, und 5 bis 14 Ringatome, vorzugsweise 5 bis 10 (insbesondere 5 oder 6) Ringatome enthält und ein oder mehrere (bevorzugt 1, 2, 3 oder 4) Sauerstoff-, Stickstoff-, Phosphor- oder Schwefel-Ringatome (bevorzugt O, S oder N) sowie Ringkohlenstoffatome enthält.

Der Ausdruck Heteroaryl bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z.B. Arylalkyl-, Arylalkenyl-, Arylalkinyl-, Arylcycloalkyl-, Arylcycloalkenyl-, Alkylaryl-cycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthalin, Indanon, Phenylcyclopentyl, Cumol, Cyclohexylphenyl, Fluoren und Indan. Bevorzugt enthält eine Aralkylgruppe ein oder zwei aromatische Ringsysteme (1 oder 2 Ringe) mit 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocyclo-alkylgruppen enthalten. Bevorzugt enthält eine Heteroaralkylgruppe ein oder zwei aromatische Ringsysteme (1 oder 2 Ringe) mit 5 oder 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocycloalkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkyl-heterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcyclo-alkyl, Heteroalkylcycloalkyl, Aryl, Heteroaryl, Aralkyl und Heteroaralkyl beziehen sich auch auf Gruppen, die gegebenenfalls substituiert sind, insbesondere auf Gruppen in denen ein oder mehrere Wasserstoffatome solcher Gruppen durch Fluor-, Chlor-, Brom- oder Jodatome oder OH, =O, SH, =S, NH₂, =NH oder NO₂-Gruppen ersetzt sind.

Der Ausdruck "gegebenenfalls substituiert" bezieht sich auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Bevorzugt sind Verbindungen der Formel (I), wobei A ein Sauerstoffatom, ein Schwefelatom, eine NH, CO, Alkylen, Alkenylen, oder Heteroalkylengruppe ist,
R¹ ein Wasserstoffatom, ein Halogenatom, eine NH₂, OH, NO₂, Alkyl, Alkenyl, Alkinyl oder Heteroalkylgruppe ist,
R^{1a} ein Wasserstoffatom, ein Halogenatom, eine NH₂, OH, NO₂, Alkyl, Alkenyl, Alkinyl oder Heteroalkylgruppe ist,
R² ein Wasserstoffatom, ein Halogenatom, eine NH₂, OH, NO₂, Alkyl, Alkenyl, Alkinyl oder Heteroalkylgruppe ist,
R^{2a} ein Wasserstoffatom, ein Halogenatom, eine NH₂, OH, NO₂, Alkyl, Alkenyl, Alkinyl oder Heteroalkylgruppe ist,
R³ ein Wasserstoffatom, ein Halogenatom, eine NH₂, OH, NO₂, Alkyl, Alkenyl, Alkinyl oder Heteroalkylgruppe ist,
R^{3a} ein Wasserstoffatom, ein Halogenatom, eine NH₂, OH, NO₂, Alkyl, Alkenyl, Alkinyl oder Heteroalkylgruppe ist,
R⁴ ein Wasserstoffatom, ein Halogenatom, eine NH₂, OH, NO₂, Alkyl, Alkenyl, Alkinyl oder Heteroalkylgruppe ist,
R^{4a} ein Wasserstoffatom, ein Halogenatom, eine NH₂, OH, NO₂, Alkyl, Alkenyl, Alkinyl oder Heteroalkylgruppe ist,
R⁵ ein Wasserstoffatom, ein Halogenatom, eine NH₂, OH, NO₂, Alkyl, Alkenyl, Alkinyl oder Heteroalkylgruppe ist, und
R^{5a} ein Wasserstoffatom, ein Halogenatom, eine NH₂, OH, NO₂, Alkyl, Alkenyl, Alkinyl oder Heteroalkylgruppe ist.

Bevorzugt sind die Reste R¹, R^{1a}, R², R^{2a}, R³, R^{3a}, R⁴, R^{4a}, R⁵ und R^{5a} unabhängig voneinander H, F, Cl, NH₂, OH, NO₂, C₁-C₄ Alkylgruppen (insbesondere CH₃ oder ^{t}butyl) oder C₁-C₄ Heteroalkylgruppen (insbesondere NHCOCH₃, OCH₃ oder CH₂OH).

Weiter bevorzugt hat die kürzeste Kette der Gruppe A zwischen den beiden Phenylringen bzw. den Ringen D und E eine Länge von ein oder zwei Atomen, ausgewählt aus C, O, S oder N, insbesondere C oder O.

Besonders bevorzugt hat die kürzeste Kette der Gruppe A zwischen den beiden Phenylringen bzw. den Ringen D und E eine Länge von einem Atom, ausgewählt aus O oder C, insbesondere C.

Weiter bevorzugt ist R¹ ein Wasserstoffatom.

Weiter bevorzugt ist R^{1a} ein Wasserstoffatom.

Weiter bevorzugt sind R¹ und R^{1a} zusammen eine Bindung.

Weiter bevorzugt ist R² ein Wasserstoffatom oder eine Gruppe der Formel NH₂, OH, CH₃, ^{t}Bu, NHCOCH₃, NO₂ oder CH₂OH, insbesondere ein Wasserstoffatom, eine OH oder eine NH₂ Gruppe, besonders bevorzugt H oder OH.

Weiter bevorzugt ist R^{2a} ein Wasserstoffatom oder eine Gruppe der Formel NH₂, OH, CH₃, ^{t}Bu, NHCOCH₃, NO₂ oder CH₂OH, insbesondere ein Wasserstoffatom, eine OH oder eine NH₂ Gruppe, besonders bevorzugt H, OH oder NH₂.

Weiter bevorzugt ist R³ ein Wasserstoffatom, ein Chloratom, ein Fluoratom oder eine Gruppe der Formel NH₂, OH oder OCH₃, insbesondere eine OH Gruppe oder ein Wasserstoffatom.

Weiter bevorzugt ist R^{3a} ein Wasserstoffatom, ein Chloratom, ein Fluoratom oder eine Gruppe der Formel NH₂, OH oder OCH₃, insbesondere eine OH Gruppe oder ein Wasserstoffatom.

Weiter bevorzugt ist R⁴ ein Wasserstoffatom oder eine Gruppe der Formel NH₂, OH, CH₃, ^{t}Bu, NHCOCH₃, NO₂ oder CH₂OH, insbesondere ein Wasserstoffatom.

Weiter bevorzugt ist R^{4a} ein Wasserstoffatom oder eine Gruppe der Formel NH₂, OH, CH₃, ^{t}Bu, NHCOCH₃, NO₂ oder CH₂OH, insbesondere ein Wasserstoffatom.

Weiter bevorzugt ist R⁵ ein Wasserstoffatom.

Weiter bevorzugt ist R^{5a} ein Wasserstoffatom.

Weiter bevorzugt sind R⁵ und R^{5a} zusammen eine Bindung.

Des Weiteren bevorzugt ist von den Resten R², R^{2a}, R⁴ und R^{4a} maximal einer eine NH₂ Gruppe, wenn R³ und R^{3a} eine OH Gruppe sind.

Weiter bevorzugt sind von den Resten R², R^{2a}, R⁴ und R^{4a} mindestens drei eine NH₂ Gruppe, wenn R³ und R^{3a} eine OH Gruppe sind.

Weiter bevorzugt sind Verbindungen der Formel (I) wobei R³ eine OH-Gruppe ist und R² und R⁴ keine NH₂ Gruppen sind, (insbesondere wobei R² ein Wasserstoffatom, eine OH, eine Alkyl oder eine Heteroalkylgruppe ist und R⁴ ein Wasserstoffatom ist).

Weiter bevorzugt sind Verbindungen der Formel (II): 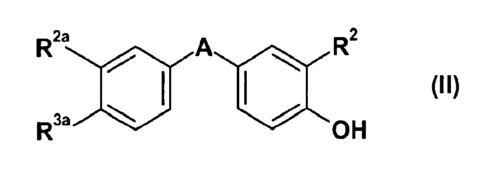 wobei R² ein Wasserstoffatom oder eine OH Gruppe ist und die Reste R^{2a} und R^{3a} sowie die Gruppe A wie oben definiert sind.

Bevorzugt sind Verbindungen der Formeln (I) oder (II) wobei A aus den folgenden Gruppen ausgewählt ist: O, S, NH, NCH₃, CH₂, CH(CH₃), C(CF₃)₂, CH(OH), CO, CH₂CH₂, oder COCH₂, insbesondere CO, CH(CH₃) und CH₂.

Besonders bevorzugt sind Verbindungen der Formel (II) wobei A aus den folgenden Gruppen ausgewählt ist: CH₂, CH(CH₃) oder CO, R² eine OH Gruppe ist und R^{2a} und R^{3a} wie oben definiert sind. Insbesondere ist dabei R^{2a} ein Wasserstoffatom oder eine OH oder NH₂ Gruppe und R^{3a} ein Wasserstoffatom oder eine OH Gruppe.

Weiterhin besonders bevorzugt sind Verbindungen der Formel (II) wobei A aus den folgenden Gruppen ausgewählt ist: CH₂, CH(CH₃) oder CO, R² ein Wasserstoffatom ist und R^{2a} und R^{3a} wie oben definiert sind. Insbesondere ist dabei R^{2a} ein Wasserstoffatom oder eine OH oder NH₂ Gruppe und R^{3a} ein Wasserstoffatom oder eine OH Gruppe.

Weiter bevorzugt sind Verbindungen der Formel (III): 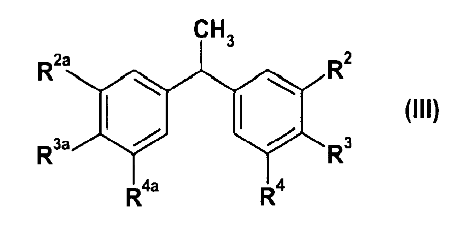 wobei die Reste R², R^{2a}, R³, R^{3a}, R⁴ und R^{4a} wie oben definiert sind.

Besonders bevorzugt sind Verbindungen der Formel (III) wobei R² ein Wasserstoffatom, eine OH oder eine NH₂ Gruppe ist, R^{2a} ein Wasserstoffatom, eine OH oder eine NH₂ Gruppe ist, R³ und R^{3a} OH Gruppen sind und R⁴ und R^{4a} Wasserstoffatome sind.

Bevorzugt sind die in den Tabellen 1, 2 und 3 offenbarten Verbindungen.

Insbesondere bevorzugt sind die folgenden Verbindungen:

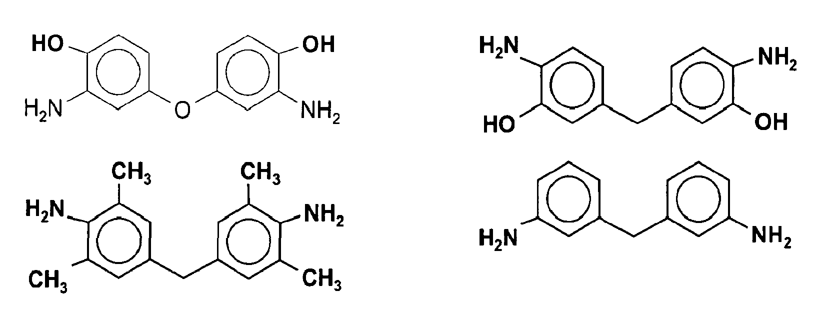

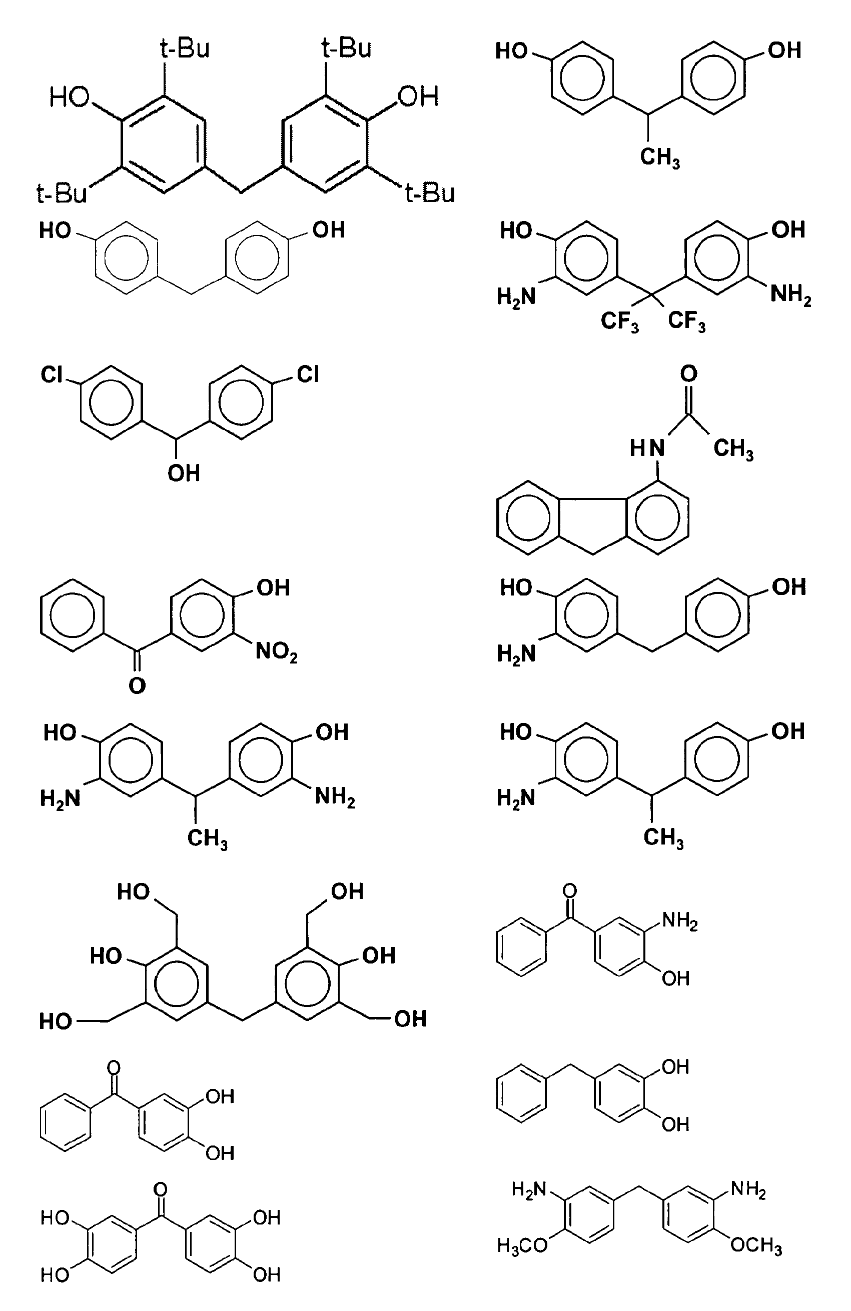

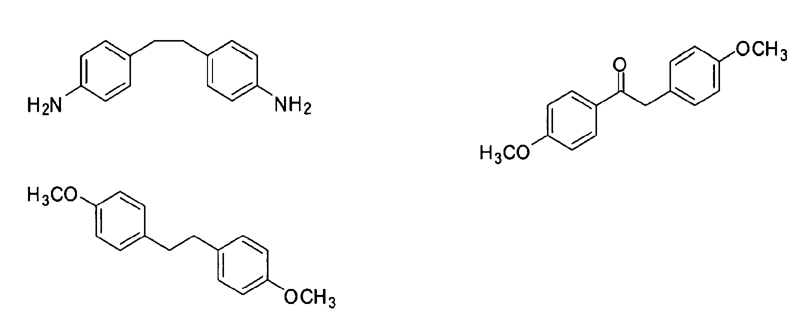

Besonders bevorzugt sind die folgenden Verbindungen:

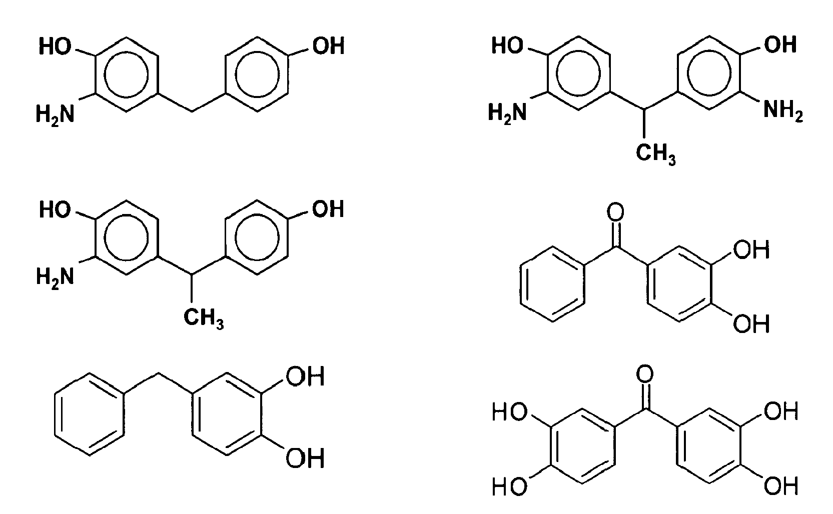

Bevorzugt sind die in WO 2006/122546, insbesondere in Tab. 2.5, Tab. A, Tab. B, Tab. C und Tab. D, offenbarten Verbindungen von der vorliegenden Erfindung ausgenommen.

Insbesondere sind die folgenden Verbindungen von der vorliegenden Erfindung ausgenommen:

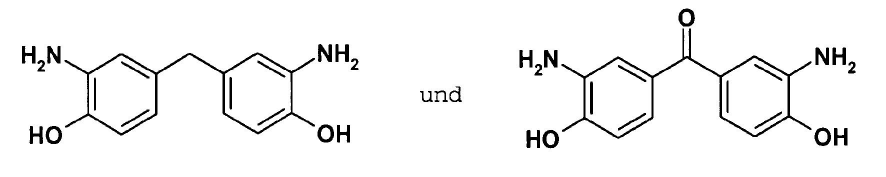

Die bevorzugten Ausführungsformen können selbstverständlich in jeder beliebigen Kombination miteinander kombiniert werden.

Die Verbindungen der vorliegenden Erfindung können zur Modifikation, insbesondere einer Inhibition einer AKAP-PKA-Interaktion verwendet werden.

Die Verbindungen der vorliegenden Erfindung können bei einer Erkrankung eingesetzt werden, die mit Fehlregulationen in kompartimentaliserter cAMP-abhängiger Signalverarbeitung assoziiert ist. Dazu gehört insbesondere eine Krankheit wie Hypertrophie des Herzens, eine koronare Herzkrankheit, Hypertonie und Herzinsuffizienz.

Die Erfindung betrifft auch die Verwendung einer Verbindung der Formel (I), (II), (III) oder (X) oder Kombinationen derselben zur spezifischen Bindung an AKAP, bevorzugt AKAP18, besonders bevorzugt AKAP18delta und/oder eine spezifische Bindung an PKA, bevorzugt Untereinheiten dieser, ganz besonders bevorzugt an RII-Untereinheiten.

Die Erfindung betrifft auch die Inhibition der Interaktion von RIα-, RIIα-, RIβ und/oder RIIβ Untereinheiten der PKA mit AKAP.

Verbindungen der Formel (I), (II), (III) oder (X) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I), (II), (III) oder (X) sowie Gemische davon umfasst. Des Weiteren sind von der vorliegenden Erfindung alle tautomeren Formen der Verbindungen der Formel (I), (II), (III) oder (X) umfasst.

Die therapeutische Verwendung der Verbindungen der Formel (I), (II), (III) oder (X), ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Die vorliegende Erfindung umfasst ferner Verbindungen der Formeln (I), (II), (III) oder (X) zur Verwendung als Arzneimittel zur Behandlung der genannten Krankheiten.

Die pharmazeutischen Zusammensetzungen gemäß der vorliegenden Erfindung enthalten mindestens eine Verbindung der Formel (I), (II), (III) oder (X) und fakultativ Trägerstoffe und/oder Adjuvantien.

Beispiele für pharmakologisch akzeptable Salze der Verbindungen der Formel (I), (II), (III) oder (X) sind Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Weitere Beispiele für pharmakologisch akzeptable Salze der Verbindungen der Formel (I), (II), (III) oder (X) sind Alkali- oder Erdalkalisalze wie z. B. Natrium, Kalium, Lithium, Calcium oder Magnesium Salze, Ammoniumsalze oder Salze von organischen Basen wie z. B. Methylamin, Dimethylamin, Triethylamin, Piperidin, Ethylendiamin, Lysin, Cholinhydroxid, Meglumin, Morpholin oder Arginin Salze. Verbindungen der Formel (I), (II), (III) oder (X) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I), (II), (III) oder (X) auftreten. Wenn die Verbindungen der Formel (I), (II), (III) oder (X) asymmetrische C-Atome enthalten, können sie entweder als achirale Verbindungen, Diastereomeren-Gemische, Gemische von Enantiomeren oder als optisch reine Verbindungen vorliegen.

Die Pro-Drugs, die ebenfalls Gegenstand der vorliegenden Erfindung sind, bestehen aus einer Verbindung der Formel (I), (II), (III) oder (X) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Ethoxy-, Benzyloxy-, Acetyl- oder Acetyloxy-Gruppe.

Auch die Verwendung dieser Wirkstoffe zur Herstellung von Arzneimitteln ist Gegenstand der vorliegenden Erfindung. Im Allgemeinen werden Verbindungen der Formel (I), (II), (III) oder (X) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere Wirkstoffe beinhalten die zur Behandlung der oben genannten Krankheiten verwendet werden können.

Zur Vorbeugung und/oder Behandlung der oben beschriebenen Erkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 1 mg bis 4000 mg pro Tag geeignet, wobei eine bevorzugte Dosis 50 bis 3000 mg pro Tag ist. In geeigneten Fällen kann die Dosis auch unter oder über den oben angegebenen Werten liegen. Die tägliche Dosis kann als einfache Gabe oder in mehrfachen Gaben verabreicht werden. Eine typische Einzeldosis beinhaltet etwa 50 mg, 100 mg, 250 mg, 500 mg, 1 g oder 2 g des Wirkstoffs.

Die erfindungsgemäßen Verbindungen können in einer bevorzugten Ausführungsform in einer Gesamtmenge von bevorzugt 0,05 bis 500mg/kg Körpergewicht je 24 Stunden eingesetzt werden, bevorzugt von 5 bis 100mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,005 bis 500mg/kg, bevorzugt von 0,05 bis 500mg/kg Körpergewicht kann einmalig oder mehrfach angewendet werden, um die gewünschten Ergebnisse zu erhalten. Typischerweise werden insbesondere pharmazeutischen Mittel zur etwa 1- bis 10-maligen Verabreichung pro Tag oder alternativ oder zusätzlich als kontinuierliche Infusion verwendet. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Tagesdosis auf 2 bis 5 Applikationen zu verteilen, wobei bei jeder Applikation zum Beispiel 1 bis 2 Tabletten mit einem Wirkstoffgehalt von 0,05 bis 500mg/kg Körpergewicht verabreicht werden. Selbstverständlich ist es möglich, den Wirkstoffgehalt auch höher zu wählen, beispielsweise bis zu einer Konzentration bis 5000mg/kg. Die Tabletten können auch retardiert sein, wodurch sich die Anzahl der Applikationen pro Tag auf 1 bis 3 vermindert. Der Wirkstoffgehalt der retardierten Tabletten kann 3 bis 3000mg betragen. Wenn der Wirkstoff - wie ausgeführt - durch eine Injektion verabreicht wird, ist es bevorzugt, 1- bis 10-mal pro Tag beziehungsweise durch Dauerinfusion den Wirt mit den erfindungsgemäßen Verbindungen in Kontakt zu bringen, wobei Mengen von 1 bis 4000mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens erfolgen.

Die Erfindung betrifft auch ein Verfahren zur Modifikation, insbesondere einer Inhibition einer AKAP-PKA Interaktion umfassend die Schritte:
(a) Bereitstellung einer erfindungsgemäßen Verbindung und
(b) In-Kontakt-Bringen mindestens einer Verbindung gemäß (a) mit einer Zelle, einer Zellkultur, einem Gewebe und/oder einem Zielorganismus.

In einer bevorzugten Ausführungsform der Erfindung ist dieses Verfahren **dadurch gekennzeichnet, dass** die Modifikation an einer regulatorischen RII-Untereinheit der PKA erfolgt, wobei besonders bevorzugt die RII-Untereinheiten R11α und/oder R11β-Untereinheiten sind.

Die Erfindung betrifft auch einen Kit, der die erfindungsgemäßen Verbindungen und/oder eine pharmazeutische Zusammensetzung gemäss der Erfindung, ggf. zusammen mit einer Information - z. B. einem Beipackzettel oder einer Internet-Adresse, die auf Homepages mit weiteren Informationen verweist etc. - über die Handhabung bzw. über die Kombination der Inhalte des Kits umfasst. Die Information zur Handhabung des Kits kann beispielsweise ein Therapie-Schema für die o. g. Krankheiten umfassen. Die Information kann jedoch auch Angaben darüber umfassen, wie die erfindungsgemäßen Erzeugnisse innerhalb einer Diagnose von Krankheiten, die mit AKAP-PKA Interaktionen oder deren Entkopplung assoziiert sind, einzusetzen sind. Der erfindungsgemäße Kit kann auch in der Grundlagenforschung verwendet werden. Innerhalb der Grundlagenforschung ist der Kit bevorzugt einsetzbar, um zu detektieren, ob ein Stoffwechselphänomen mit der Wecheslwirkung bzw. mit der nicht vorhandenen Wechselwirkung von AKAP und PKA assoziiert ist. Insbesondere ist es möglich, mithilfe des erfindungsgemäßen Kits zu bestimmen, welche Untereinheiten von AKAP und/oder PKA für die Interaktion dieser beiden Moleküle oder für das Nichtzustandekommen der Interaktion zwischen diesen verantwortlich sind.

Verbindungen der Formeln (I), (II) und (III) sind z. T. kommerziell erhältlich bzw. können nach den unten beschriebenen Verfahren hergestellt werden:

### Synthesebeispiele

### Synthese von 4-Benzyl-pyrocatechol (Verbindung 1027)

100 mg 3,4-Dihydroxybenzophenon werden in 10 mL Methanol gelöst, mit 20 mg 10% Pd/C versetzt und 6 Stunden unter Wasserstoff-Atmosphäre bei Raumtemperatur gerührt. Nach 6 Stunden ist die Reaktion vollständig durchgelaufen (TLC-Kontrolle, Petrolether:Essigester = 2:1). Pd/C wird abfiltriert und mit Methanol gewaschen. Nach dem Abdestillieren der Lösungsmittel am Rotationsverdampfer erfolgt die Reinigung mittels Flash-Chromatographie (Petrolether:Essigester = 4:1). Ausbeute: 81 mg, grauer Feststoff; MS (ESI): M+Na⁺ = 223.0, M⁻ = 199.1; Reinheit = 97.5%; UV: λₘₐₓ = 285 nm. C₁₃H₁₂O₂

### Synthese von Bis-(3,4-dihydroxy-phenyl)-methanon (Verbindung 1028)

11 g Phosphorpentoxid werden mit 8 mL 85 % Phosphorsäure vermischt und für 1 Stunde bei +85 °C erhitzt. Die Mischung aus 1 g (6.5 mmol, 1 Äquiv.) 3,4-Dihydroxy-benzoesäure (Fluka, 37580) und 0.79 g (7.1 mmol, 1.1 Äquiv.) Brenzkatechin (Fluka, 15880) wird zugegeben. Das Reaktionsgemisch wird eine weitere Stunde bei dieser Temperatur gerührt (die Reaktion wird mittels TLC (PE:EE = 1:2) kontrolliert. Die wässrige Phase wird mit Ethylacetat (3x50 mL) extrahiert und die vereinigten organischen Extrakte werden über MgSO₄ getrocknet. Nach dem Abdestillieren der Lösungsmittel am Rotationsverdampfer erfolgt die Reinigung mittels Flash-Chromatographie (Petrolether:Essigester = 2:1). Das Rohprodukt wird in Methanol gelöst und mit 5 g Kieselgel versetzt, das Lösungsmittel im Vakuum bis zur Trockne abrotiert und so auf eine Säule aufgetragen. Ausbeute: 700 mg, farbloser Feststoff; MS (ESI): M+Na⁺ = 269.0, M⁻ = 245.1; Reinheit = 99.7%; UV: λₘₐₓ = 267 nm. C₁₃H₁₀O₅. Originalvorschrift: D. C. Ayres and R. C. Denney, J. Chem. Soc., 1961, 4506 - 4509.

### Synthese von 1,1-Bis-(3-Amino-4-hydroxyphenyl)-ethan und 3-Amino-1,1-bis-(4-hydroxyphenyl)-ethan (Verbindungen 1020 und 1021)

Zu 5 mL einer verdünnten Lösung von Salpetersäure (1 mL 65% HNO₃ in 4 ml Wasser) werden bei 0 °C 300 mg 4,4'-Ethylidenbisphenol zugegeben. Das Wasserbad wird entfernt und das Reaktionsgemisch zuerst 20 Stunden bei Raumtemperatur gerührt, dann mit 3 mL Acetonitril versetzt und weitere 24 Stunden bei Raumtemperatur gerührt. Der gelbe Feststoff wird abfiltriert, mit Wasser gewaschen und an der Luft getrocknet. Das so erhaltene Zwischenprodukt wird ohne Reinigung in der nächsten Reaktion umgesetzt. Es wird in 10 mL trockenem Methanol suspendiert, mit 30 mg 10% Pd/C versetzt und 24 Stunden unter Wasserstoff-Atmosphäre bei Raumtemperatur gerührt. Nach dieser Zeit ist die Reaktion vollständig durchgelaufen (HPLC-Kontrolle, 30% Acetonitril, 1 mL/min, 284 nm). Pd/C wird abfiltriert und mit Methanol gewaschen. Nach dem Abdestillieren der Lösungsmittel am Rotationsverdampfer erfolgt die Reinigung mittels Flash-Chromatographie (Dichlormethan:Methanol = 15:1, dann 10:1). Ausbeute für **1020:** 106 mg, grauer Feststoff; MS (ESI): M⁺ = 245.1, M⁻ = 242.8; Reinheit = 96.6%; UV: λₘₐₓ = 292 nm; Ausbeute für **1021:** 28 mg, grauer Feststoff; MS (ESI) : M⁺ = 229.9, M⁻ = 227.8; Reinheit = 95.1%; UV: λₘₐₓ = 288 nm.

### Synthese von 3-Amino-4,4'-dihydroxydiphenylmethan und 3,3'-Diamino-4,4'-dihydroxydiphenylmethan (Verbindungen 1016 und 18882)

Zu 60 mL einer verdünnten Lösung von Salpetersäure (12 ml 65% HNO₃ in 48 ml Wasser) werden bei 0 °C 5 g Bis-(4-hydroxyphenylmethan) zugegeben. Das Wasserbad wird entfernt und das Reaktionsgemisch zuerst 20 Stunden bei Raumtemperatur gerührt, dann mit 15 mL Acetonitril versetzt und weitere 48 Stunden bei Raumtemperatur gerührt. Der gelbe Feststoff wird abfiltriert und bei 60 °C im Vakuum über mehrere Stunden getrocknet. Das so erhaltene Zwischenprodukt wird ohne Reinigung in der nächsten Reaktion umgesetzt. Es wird in 50 mL trockenem Methanol suspendiert, mit 550 mg 10% Pd/C versetzt und 48 Stunden unter Wasserstoff-Atmosphäre bei Raumtemperatur gerührt. Nach dieser Zeit ist die Reaktion vollständig durchgelaufen (HPLC-Kontrolle, 60% Acetonitril, 1 mL/min, 270 nm). Pd/C wird abfiltriert und mit Methanol gewaschen. Nach dem Abdestillieren der Lösungsmittel am Rotationsverdampfer erfolgt die Reinigung von 500 mg des Restes mittels Flash-Chromatographie (Dichlormethan:Methanol = 15:1, dann 10:1). Ausbeute für **1016:** 67 mg, grauer Feststoff; MS (ESI): M⁺ = 216.1, M⁻ = 213.8; Reinheit = 96.2%; UV: λₘₐₓ = 289 nm; C₁₃H₁₃NO₂; Ausbeute für **18882:** 294 mg, grauer Feststoff; MS (ESI): M⁺ = 231.1, M⁻ = 228.8; Reinheit = 99.4%; UV: λₘₐₓ = 293 nm; C₁₃H₁₄N₂O₂.

### Synthese von Bis-(3-amino-4-hydroxy-phenyl)-methan (Verbindung 1030)

25 mg Bis-(4-methoxy-3-nitro-phenyl)-methanon werden in 10 mL Methanol gelöst, mit 5 mg 10% Pd/C versetzt und 18 Stunden unter Wasserstoff-Atmosphäre bei Raumtemperatur gerührt. Nach dieser Zeit ist die Reaktion vollständig durchgelaufen (TLC-Kontrolle, Petrolether:Essigester=2:1). Pd/C wird abfiltriert und mit Methanol gewaschen. Nach dem Abdestillieren der Lösungsmittel am Rotationsverdampfer erfolgt die Reinigung mittels Flash-Chromatographie (Petrolether:Essigester = 2:1). Ausbeute: 23 mg, farbloser Feststoff; MS (ESI): M⁺ = 259.1; Reinheit = 98.2%; UV: λₘₐₓ = 293 nm; C₁₅H₁₈N₂O₂.

Für die Reinheitskontrolle aller Substanzen wird folgendes HPLC-System aus Wasser und Acetonitril benutzt: Reversed Phase Kieselgel (RP-18), Gradient von 100% Wasser bis auf 100% Acetonitril in 60 Minuten, Flow 1,5 ml/min.; UV-Detektion mit var. Wellenlänge.

### Synthese von Bis-(3,4-dihydroxy-phenyl)-methanon (#1028)

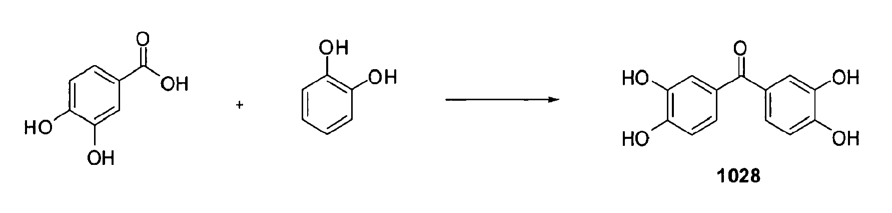

11 g Phosphorpentoxid werden mit 8 mL 85 % Phosphorsäure vermischt und für 1 Stunde bei +85 °C erhitzt. Die Mischung aus 1 g (6.5 mmol, 1 Äquiv.) 3,4-Dihydroxy-benzoesäure (Fluka, 37580) und 0.79 g (7.1 mmol, 1.1 Äquiv.) Brenzkatechin (Fluka, 15880) wird zugegeben. Das Reaktionsgemisch wird eine weitere Stunde bei dieser Temperatur gerührt (Kontrolle mittels TLC (PE:EE = 1:2)) und anschließend in 50 mL Eiswasser gegossen (Achtung, eventuell ist das Erhitzen über einen noch längeren Zeitraum sinnvoller, damit die ganze 3,4-Dihydroxy-benzoesäure abreagiert; es erleichtert später die Flash-Chromatographie). Die wässrige Phase wird mit Ethylacetat (3x50 mL) extrahiert und die vereinigten organischen Extrakte werden über MgSO₄ getrocknet. Nach dem Abdestillieren der Lösungsmittel am Rotationsverdampfer erfolgt die Reinigung mittels Flash-Chromatographie (Petrolether:Essigester = 2:1). Ausbeute: 700 mg. MS (ESI): M+Na⁺ = 269.0, M⁻ = 245.1; Reinheit = 99.7%; UV: λₘₐₓ = 267 nm. C₁₃H₁₀O₅.

Dazu wurde das Rohprodukt in Methanol gelöst und mit 5 g Kieselgel versetzt, das Lösungsmittel im Vakuum bis auf Trocken abrotiert und so auf eine Säule aufgetragen.

Originalvorschrift: D. C. Ayres and R. C. Denney, J. Chem. Soc., 1961, 4506-4509.

Die Untersuchungen zur Aktivität der vorliegenden Verbindungen der Formeln (I), (II) oder (III) wurden z. T. wie in WO 2006/122546 beschrieben durchgeführt.

### Tabellen

In den Tabellen 1, 2 und 3 sind bevorzugte Verbindungen gemäß der vorliegenden Erfindung sowie die Referenzverbindung 18882 gezeigt.

### Abbildungen

### Abb. 1

Schematische Darstellung eines Proteinkinase A-Ankerproteins (AKAP) mit verschiedenen Domänen. Die Bindungsdomäne für die regulatorischen Untereinheiten der PKA (RBD, RII-Bindedomäne) ist in allen bisher identifizierten AKAPs gefunden worden (konserviert amphipathische Helix). Die für die Verankerung an subzelluläre Kompartimente verantwortliche sog. targeting-Domäne und Domänen, an die weitere Signalmoleküle (Proteine wie Phosphatasen, Kinasen oder Phosphodiesterasen) binden können (*docking*-Domäne), sind spezifisch für jedes AKAP. Dagegen enthalten nur wenige AKAP eine Domäne mit katalytischer Aktivität (z. B. RhoGEF-Aktivität), die ein Substrat umsetzen kann. R und C, regulatorische und katalytische Untereinheiten.

### Abb. 2

Die β-Adrenozeptor-vermittelte Zunahme der Kontraktilität von Kardiomyozyten. Die Stimulation von β-Adrenorezeptoren in der Plasmamembran (Sarcolemma) von Kardiomyozyten führt zur Aktivierung von PKA, die L-Typ-Ca²⁺-Kanäle im Sarcolemma und den Ryanodinrezeptor 2 (RyR₂) in der Membran des sarcoplasmatischen Retikulums (SR) phosphoryliert. Die Phosphorylierungen führen zu einem Anstieg des zytosolischen Ca²⁺. Außerdem phosphoryliert PKA Phospholamban (PLB). Die Phosphorylierung induziert die Dissoziation und dadurch die Aktivierung der Ca²⁺-ATPase SERCA2. SERCA2 kann dann effizient Ca²⁺ in das SR zurückpumpen. Insgesamt steigt durch die PKA-Phosphorylierungen die Kontraktilität der Kardiomyzyten an (positiv inotrope Wirkung). AKAP interagieren direkt mit L-Typ-Ca²⁺-Kanälen, RyR₂ und PLB und ermöglichen durch die Verankerung der PKA an diesen Substraten deren PKA-Phosphorylierung. AKAP18α, auch als AKAP15 bezeichnet, verankert die PKA direkt an L-Typ-Ca²⁺-Kanälen, mAKAP an RyR₂ und die Arbeitsgruppe des Antragstellers konnte kürzlich zeigen, dass AKAP18δ die PKA an PLB bindet.

### Abb. 3.

Schematische Darstellung des ELISA zur quantitativen Detektion der Protein-Protein-Bindung zwischen PKA-RIIα oder PKA-RIIß und GST-AKAP18δ (Glutathion S-Transferasefusion mit AKAP18δ). Die Interaktion erfolgt in Ab- oder Anwesenheit niedermolekularer Substanzen in 384-Loch-ELISA-Platten. Der Nachweis der Interaktion der Proteine erfolgt mit primären anti-AKAP18δ- und sekundären Peroxidase-gekoppelten Antikörpern und Chemilumineszenz.

### Abb. 4.

ELISA basiertes Durchmustern einer Bibliothek mit niedermolekularen Substanzen nach Hemmern der Interaktion von AKAP18δ mit regulatorischen RII-Untereinheiten der PKA: Validierung von "hits".

### Abb. 5

Die Verbindungen 18882 (FMP-API-1), 1016, 1020 und 1021 (siehe Tabelle 1) hemmen die Interaktionen von regulatorischen RII-Untereineinheiten der PKA (RIIα und β) mit AKAP150, dem AKAP Yotiao und AKAP18δ. Es wurden cAMP-Agarose-Präzipitationen mit neonatalen Kardiomyozyten in Ab- oder Anwesenheit des AKAP-PKA-Interaktionshemmers FMP-API-1 (100 µM), einer Kontrollsubstanz (100 µM) und cAMP (50 mM) durchgeführt. cAMP-Agarose bindet regulatorische Untereinheiten der PKA und deren Bindungspartner, inklusive AKAP. Präzipitierte AKAP wurden mit spezifischen Antikörpern mittels Western Blot detektiert.

### Abb. 6

Die Substanz 1016 hemmt zeitabhängig die Bindung von RIIα an Peptide, die aus der PKA-Bindedomäne von AKAP18δ abgeleitet sind (AKAP18δ-L314E). Die RIIα -Bindung an das Peptid AKAP18δ-L314E (Hundsrucker et al., Biochem. J. 2006) wird durch die Substanz 1016 inhibiert. Eine Präinkubation von RIIα für 2 h hemmt die Interaktion um etwa 50 %, also effizienter als 18882. Für das Experiment wurden Oberflächen mit dem Peptid beschichtet, mit RIIα-Untereinheiten inkubiert und die Reaktion in Biacore-Messungen verfolgt.

### Abb. 7

Die Substanz 1016 hemmt konzentrationsabhängig die Bindung von RIIα an Peptide, die aus der PKA-Bindedomäne von AKAP18δ abgeleitet sind (AKAP18δ-L314E). Die RIIα-Bindung an das Peptid AKAP18δ-L314E (Hundsrucker et al., Biochem. J. 2006) wird durch die Substanz 1016 inhibiert. Eine maximale Hemmung von etwa 60 % wird bei einer Konzentration von 100 µM erreicht, also effizienter als 18882. Für das Experiment wurden Oberflächen mit dem Peptid beschichtet, mit RIIα-Untereinheiten inkubiert und die Reaktion in Biacore-Messungen verfolgt.

### Abb. 8

Die Substanz 1020 hemmt zeitabhängig die Bindung von RIIα an Peptide, die aus der PKA-Bindedomäne von AKAP18δ abgeleitet sind (AKAP18δ-L314E). Die RIIα -Bindung an das Peptid AKAP18δ-L314E (Hundsrucker et al., Biochem. J. 2006) wird durch die Substanz 1020 inhibiert. Eine Präinkubation von RIIα für 2 h hemmt die Interaktion um etwa 50 %. Für das Experiment wurden Oberflächen mit dem Peptid beschichtet, mit RIIα-Untereinheiten inkubiert und die Reaktion in Biacore-Messungen verfolgt.

### Abb. 9

Die Substanz 1020 hemmt konzentrationsabhängig die Bindung von RIIα an Peptide, die aus der PKA-Bindedomäne von AKAP18δ abgeleitet sind (AKAP18δ-L314E). Die RIIα-Bindung an das Peptid AKAP18δ-L314E (Hundsrucker et al., Biochem. J. 2006) wird durch die Substanz 1020 inhibiert. Eine maximale Hemmung von etwa 50 % wird bei einer Konzentration von 100 µM erreicht, also effizienter als 18882. Für das Experiment wurden Oberflächen mit dem Peptid beschichtet, mit RIIα-Untereinheiten inkubiert und die Reaktion in Biacore-Messungen verfolgt.

### Abb. 10

Die Substanz 1021 hemmt zeitabhängig die Bindung von RIIα an Peptide, die aus der PKA-Bindedomäne von AKAP18δ abgeleitet sind (AKAP18δ-L314E). Die RIIα -Bindung an das Peptid AKAP18δ-L314E (Hundsrucker et al., Biochem. J. 2006) wird durch die Substanz 1021 inhibiert. Eine Präinkubation von RIIα für 2 h hemmt die Interaktion um etwa 40 %. Für das Experiment wurden Oberflächen mit dem Peptid beschichtet, mit RIIα-Untereinheiten inkubiert und die Reaktion in Biacore-Messungen verfolgt.

### Abb. 11

Die Substanz 1021 hemmt konzentrationsabhängig die Bindung von RIIα an Peptide, die aus der PKA-Bindedomäne von AKAP18δ abgeleitet sind (AKAP18δ-L314E). Die RIIα-Bindung an das Peptid AKAP18δ-L314E (Hundsrucker et al., Biochem. J. 2006) wird durch die Substanz 1021 inhibiert. Eine maximale Hemmung von etwa 50 % wird bei einer Konzentration von 100 µM erreicht, also effizienter als 18882. Für das Experiment wurden Oberflächen mit dem Peptid beschichtet, mit RIIα-Untereinheiten inkubiert und die Reaktion in Biacore-Messungen verfolgt.

### Abb. 12

Zusammenfassung: Die Substanzen 1016, 1020 und 1021 hemmen zeitabhängig die Bindung von RIIα an Peptide, die aus der PKA-Bindedomäne von AKAP18δ abgeleitet sind (AKAP18δ-L314E). Die RIIα-Bindung an das Peptid AKAP18δ-L314E (Hundsrucker et al., Biochem. J. 2006) wird durch die Substanz 1016 am effizientesten inhibiert. Eine maximale Hemmung von etwa 50 % wird nach 1 h Präinkubation erreicht. Die Sterne zeigen statistisch signifikante Unterschiede zum Ausgangswert. Für das Experiment wurden Oberflächen mit dem Peptid beschichtet, mit RIIα-Untereinheiten inkubiert und die Reaktion in Biacore-Messungen verfolgt.

### Abb. 13

Zusammenfassung: Die Substanzen 1016, 1020 und 1021 hemmen konzentrationsabhängig die Bindung von RIIα an Peptide, die aus der PKA-Bindedomäne von AKAP18δ abgeleitet sind (AKAP18δ-L314E). Die RIIα-Bindung an das Peptid AKAP18δ-L314E (Hundsrucker et al., Biochem. J. 2006) wird durch die Substanz 1016 am effizientesten inhibiert. Eine maximale Hemmung von etwa 60 % wird bei einer Konzentration von 100 µM erreicht, also effizienter als mit 18882. Die Sterne zeigen statistisch signifikante Unterschiede zum Ausgangswert. Für das Experiment wurden Oberflächen mit dem Peptid beschichtet, mit RIIα-Untereinheiten inkubiert und die Reaktion in Biacore-Messungen verfolgt.

### Abb. 14

Biacore-Messungen zum Nachweis der Bindung der Substanz 1026 an RIIα. Es wurden Oberflächen durch kovalente Bindung mit RIIα beschichtet und mit der Substanz in den angegebenen Konzentrationen inkubiert.

### Abb. 15

Biacore-Messungen zum Nachweis der Bindung der Substanz 1027 an RIIα. Es wurden Oberflächen durch kovalente Bindung mit RIIα beschichtet und mit der Substanz in den angegebenen Konzentrationen inkubiert.

### Abb. 16

Biacore-Messungen zum Nachweis der Bindung der Substanz 1028 an RIIα. Es wurden Oberflächen durch kovalente Bindung mit RIIα beschichtet und mit der Substanz in den angegebenen Konzentrationen inkubiert.

### Abb. 17

Die Bindungen des Peptids Ht31 und der Substanz 1026 an RIIα sind additiv. Es wurden Oberflächen durch kovalente Bindung mit RIIα beschichtet und mit dem Peptid Ht31, das an die sog. Dimerisierungs- und docking-Domäne (DD-Domäne) von regulatorischen Untereinheiten der PKA bindet, inkubiert.

Zusätzlich wurde mit der Substanz 1026 inkubiert. Die Bindungen sind additiv. Daher müssen die Substanzen an unterschiedliche Regionen der RII-Untereinheiten binden.

### Abb. 18

Die Bindungen des Peptids Ht31 und der Substanz 1027 an RIIα sind additiv. Es wurden Oberflächen durch kovalente Bindung mit RIIα beschichtet und mit dem Peptid Ht31, das an die sog. Dimerisierungs- und docking-Domäne (DD-Domäne) von regulatorischen Untereinheiten der PKA bindet, inkubiert. Zusätzlich wurde mit der Substanz 1027 inkubiert. Die Bindungen sind additiv. Daher müssen die Substanzen an unterschiedliche Regionen der RII-Untereinheiten binden.

### Abb. 19

Die Bindungen des Peptids Ht31 und der Substanz 1028 an RIIα sind additiv. Es wurden Oberflächen durch kovalente Bindung mit RIIα beschichtet und mit dem Peptid Ht31, das an die sog. Dimerisierungs- und docking-Domäne (DD-Domäne) von regulatorischen Untereinheiten der PKA bindet, inkubiert. Zusätzlich wurde mit der Substanz 1028 inkubiert. Die Bindungen sind additiv. Daher müssen die Substanzen an unterschiedliche Regionen der RII-Untereinheiten binden.

### Abb. 20

Biacore-Messungen zum Nachweis der konzentrationsabhängigen Bindung der Substanzen 1027, 1028 und 1030 an RIIα. Es wurden Oberflächen durch kovalente Bindung mit RIIα beschichtet und mit den Substanzen inkubiert.

### Abb. 21

Biacore-Messungen zum Nachweis der konzentrationsabhängigen Bindung der Substanzen 1025, 1026, 1027, 1028 und 1030 an RIIα. Es wurden Oberflächen durch kovalente Bindung mit RIIα beschichtet und mit den Substanzen inkubiert.

### Abb. 22

Biacore-Messungen zum Nachweis der Bindung der Substanzen, 1026, 1027, 1028, 1030 und des Peptids Ht31 (1 und 2 entspricht zwei Messungen) an RIIα. Die durchgezogene Linie entspricht dem Mittelwert der ungehemmten Bindung von RIIα an AKAP18δ L314E (n = 52) abzüglich der dreifachen Standardabweichung.

### Abb. 23

Biacore-Messungen zum Nachweis der Bindung der Substanzen 1026, 1027, und 1028 an RIIα.

### Zusammenfassung

- Die Derivate 1016, 1020 und 1021 hemmen die Bindung von RIIα an die PKA-Bindedomäne von AKAP18δ zeit- und konzentrationsabhängig effizienter und binden schneller an RIIα als die Ausgangssubstanz 18882. Die Hemmung beträgt etwa 50 %.
- Die Bindung der Substanzen an RIIα-Untereinheiten erfolgt C-terminal der Aminosäure 87, sie wirken damit anders als die sog. PKA-anchoring disruptor-Peptide (z. B. Ht31 oder AKAP18δ-L314E), die an die DD-Domäne von RII-Untereinheiten binden. Die Wirkung der Substanzen und Ht31 auf die Interaktion von RIIα mit der PKA-Bindedomäne von AKAP18δ ist daher additiv.
- Die Substanzen hemmen nicht die Bindung von RIIα-haltigem PKA-Holoenzym an die PKA-Bindedomäne von AKAP18δ. Die Daten zeigen, dass die Substanzen durch einen allosterischen Effekt die Interaktion hemmen. Die Bindung der Substanzen an RIIα hängt von der Konformation von RIIα ab.
- Befunde aus NMR-Experimenten unterstützen diese Schlussfolgerung.

### Abb. 24

Die Verbindungen 18882, 1016, 1020 und 1021 induzieren die Oligomerisierung (Oligomers) von AKAP18δ und regulatorischen RIIα- und RIIβ-Untereinheiten. Neonatale Kardiomyozyten wurden mit den Substanzen in den angegebenen Konzentrationen inkubiert. A. Anschließend wurde eine cAMP-Agarose-Präzipitation und ein Western Blot (WB) zur Detektion von AKAP18δ durchgeführt. Als Negativkontrolle wurde die cAMP-Agarose mit cAMP inkubiert, das die Bindung von R-Untereinheiten an die Agarose verhindert. B. Detektion von RIIβ in Lysaten von neonatalen Kardiomyozyten. C. Detektion von AKAP18δ, RIIα und RIIβ nach Inkubation mit den Substanzen.

### Abb. 25

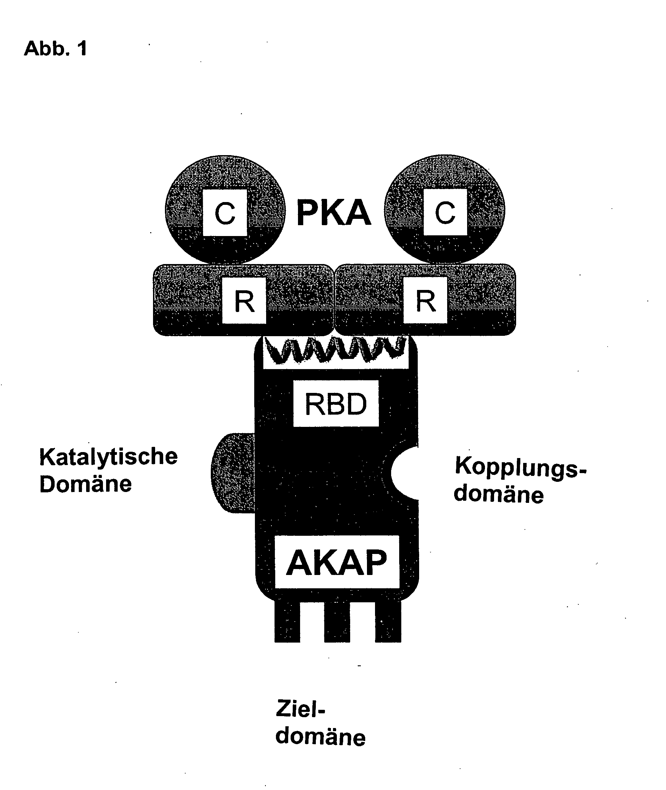

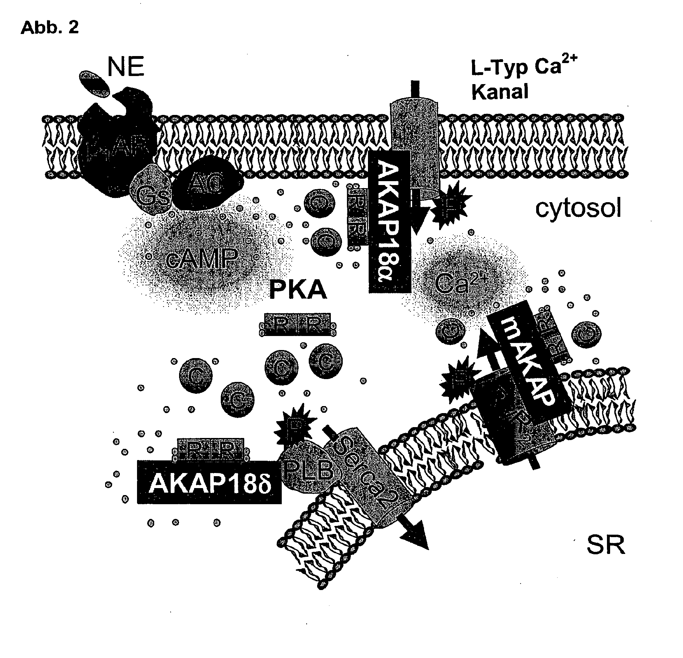

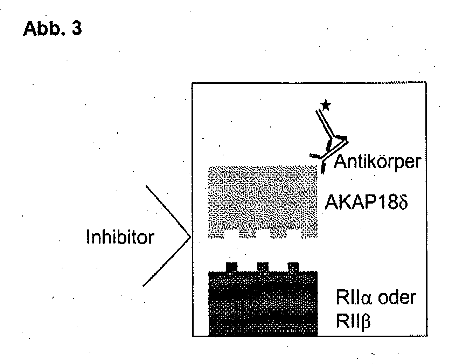

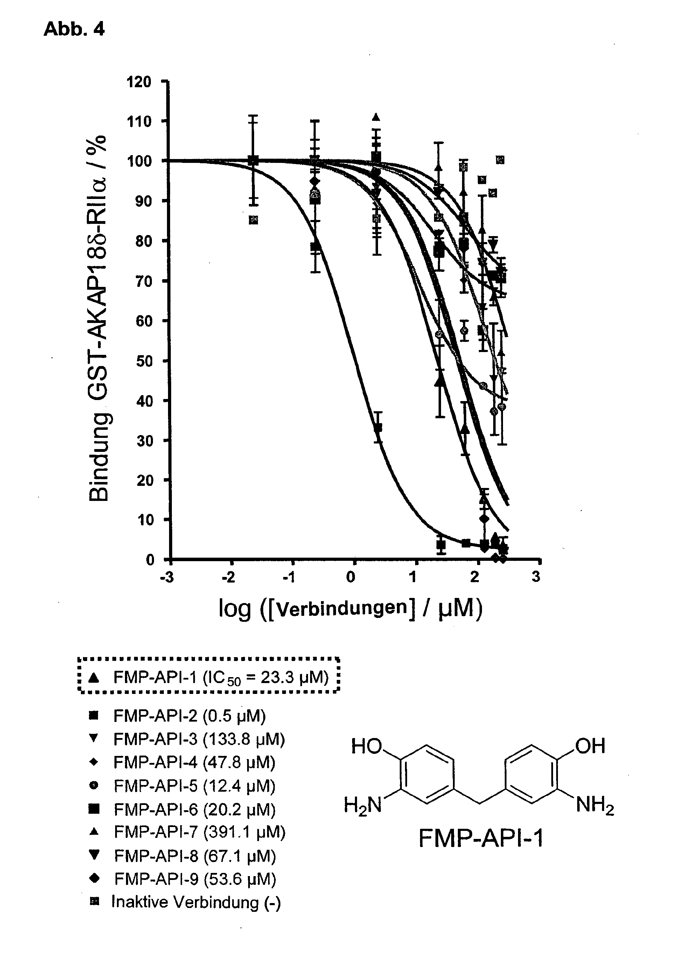

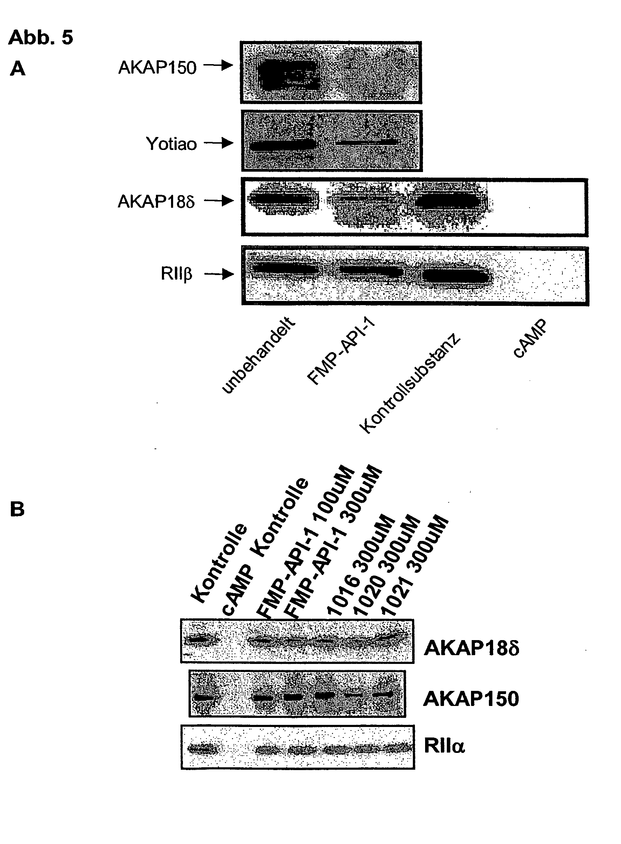

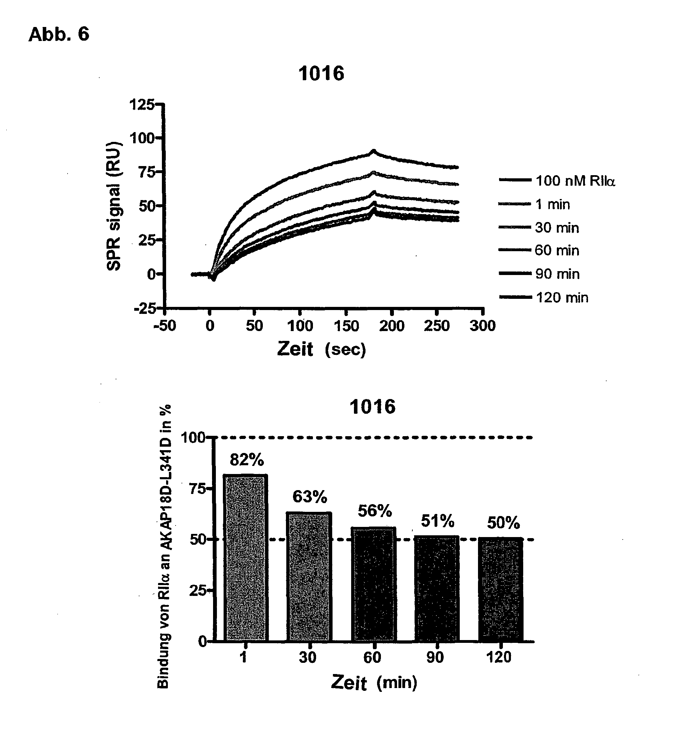

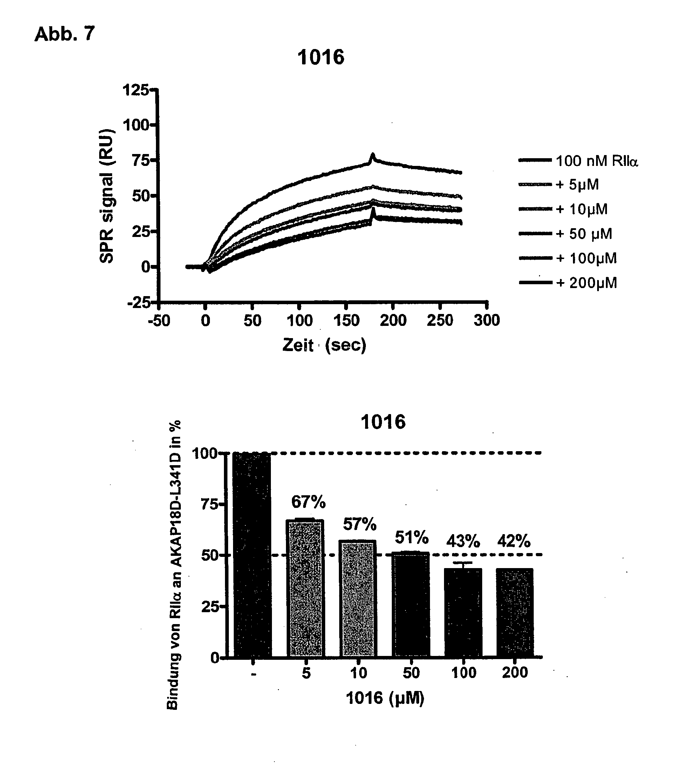

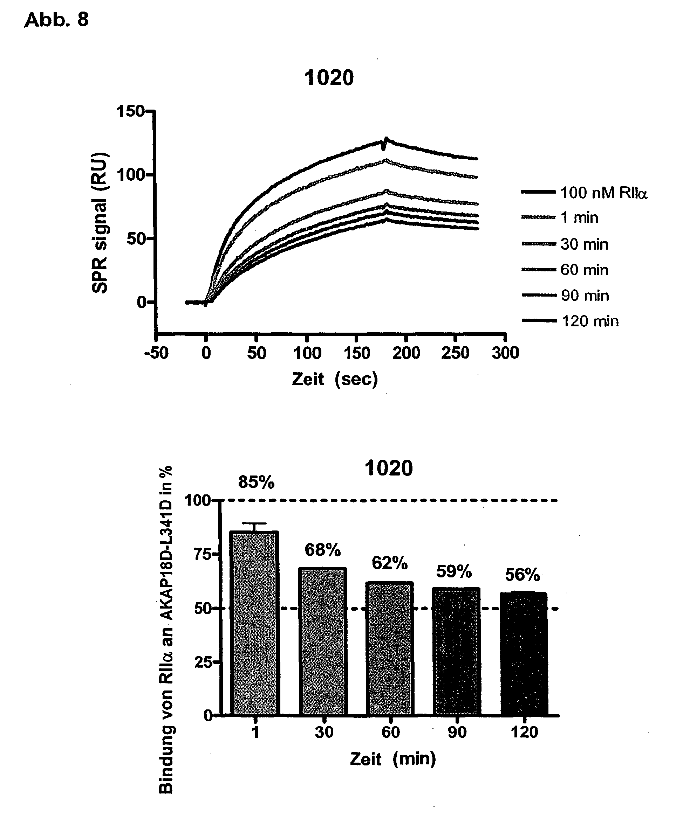

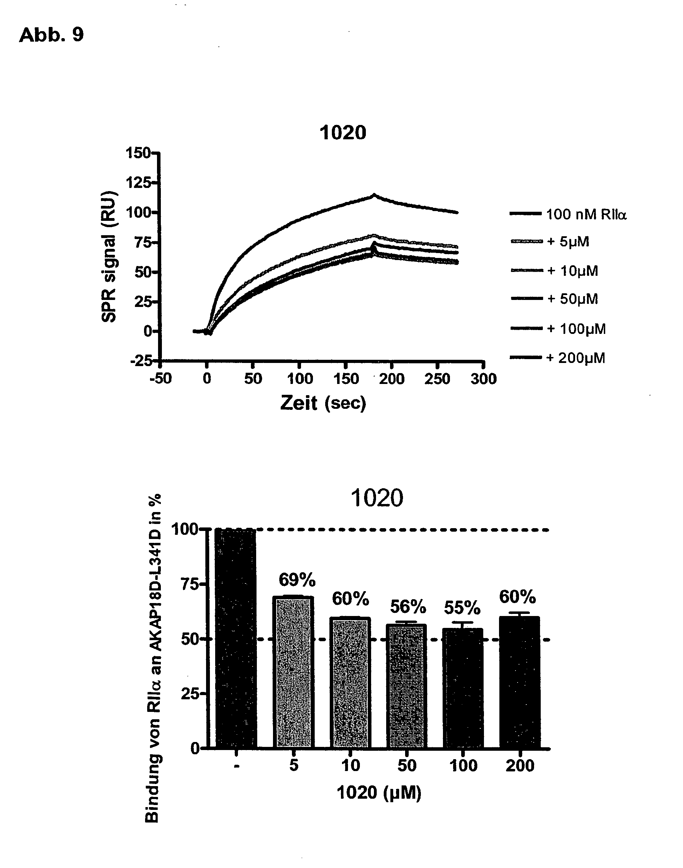

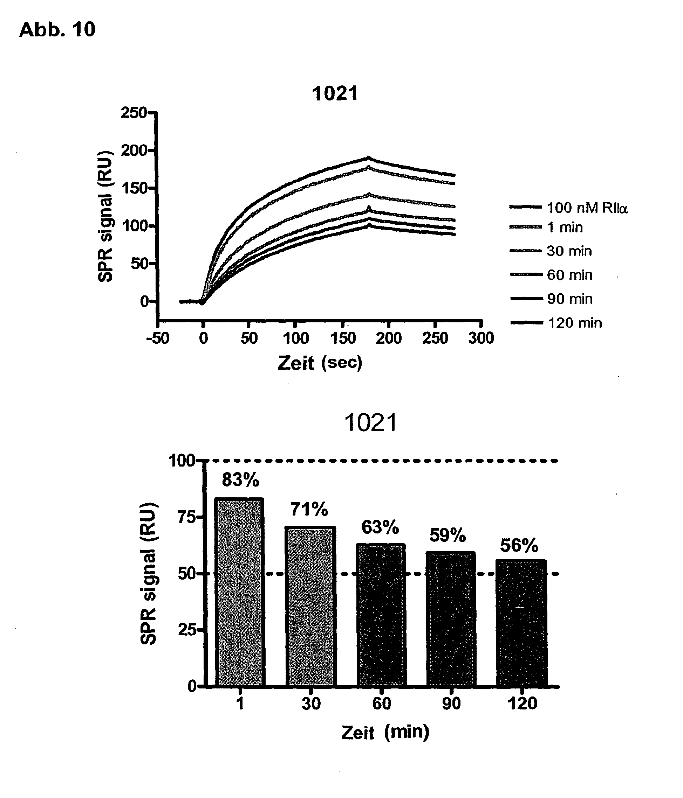

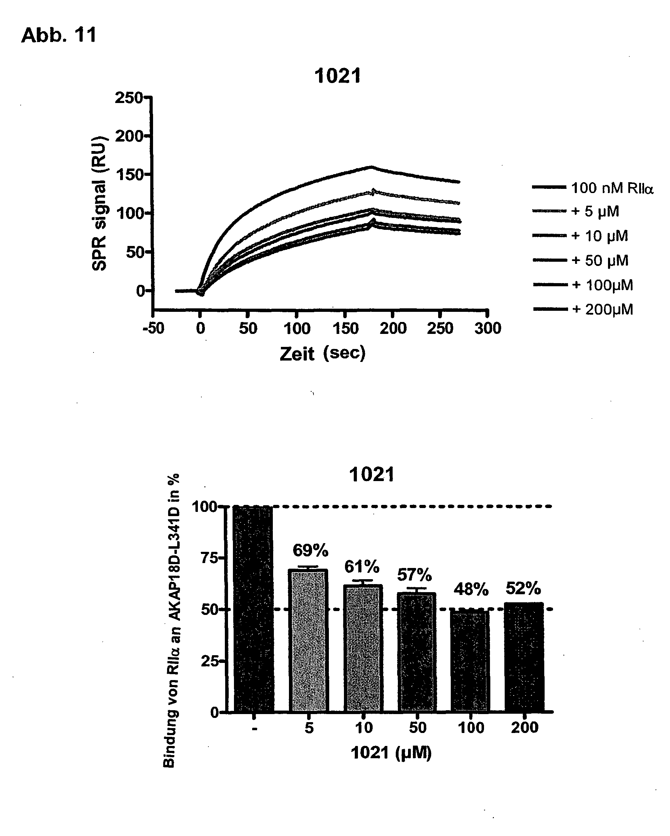

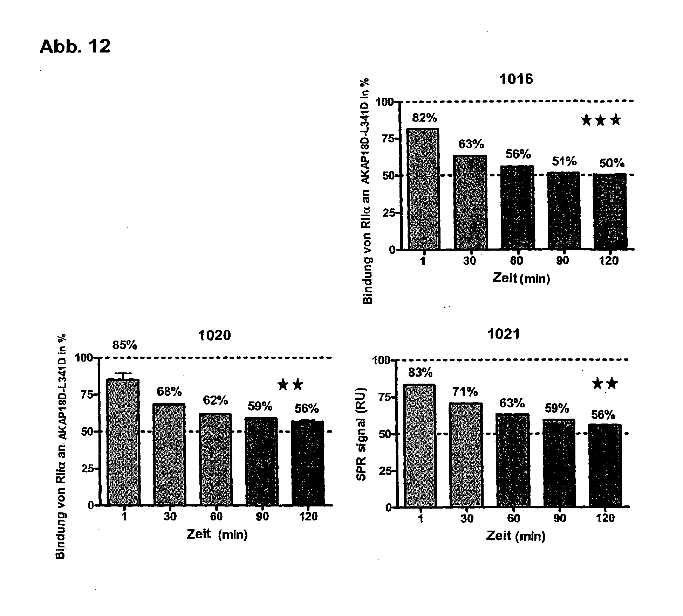

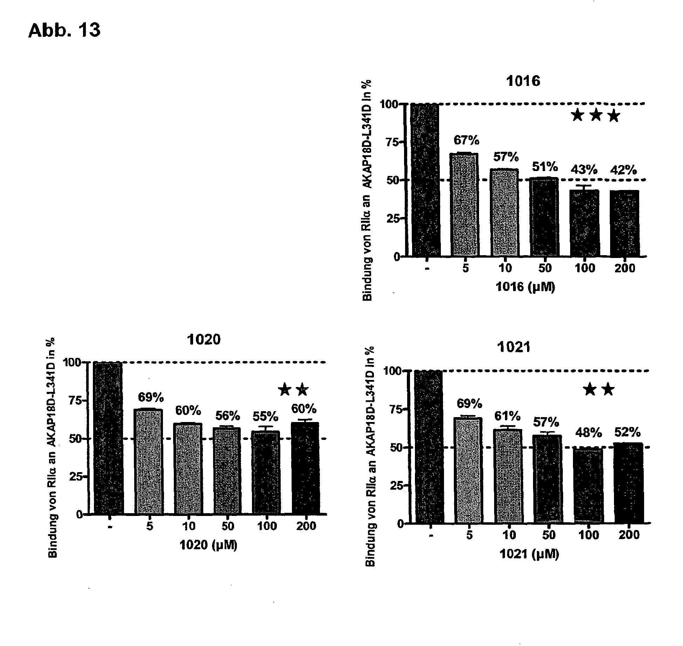

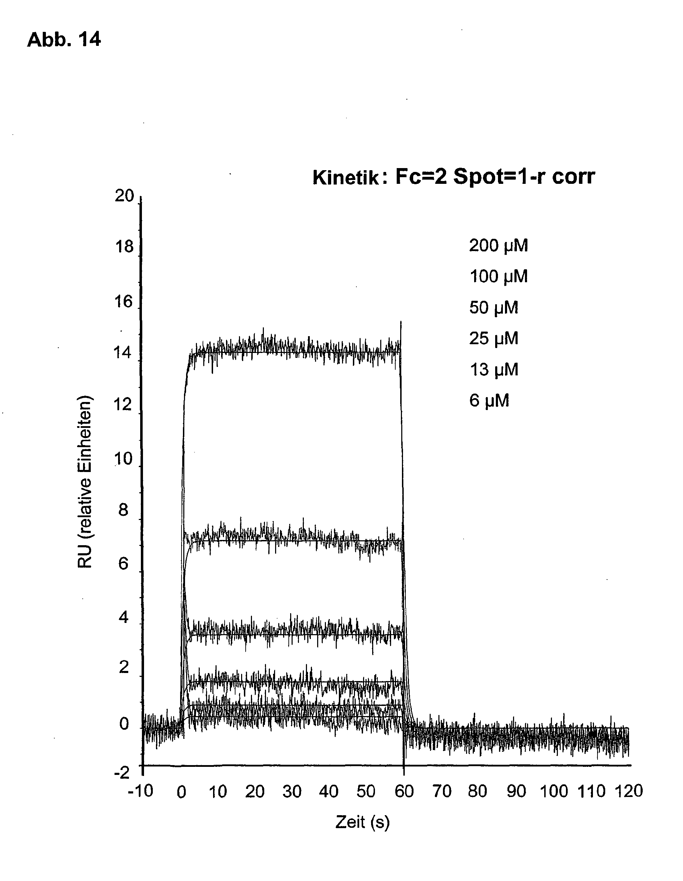

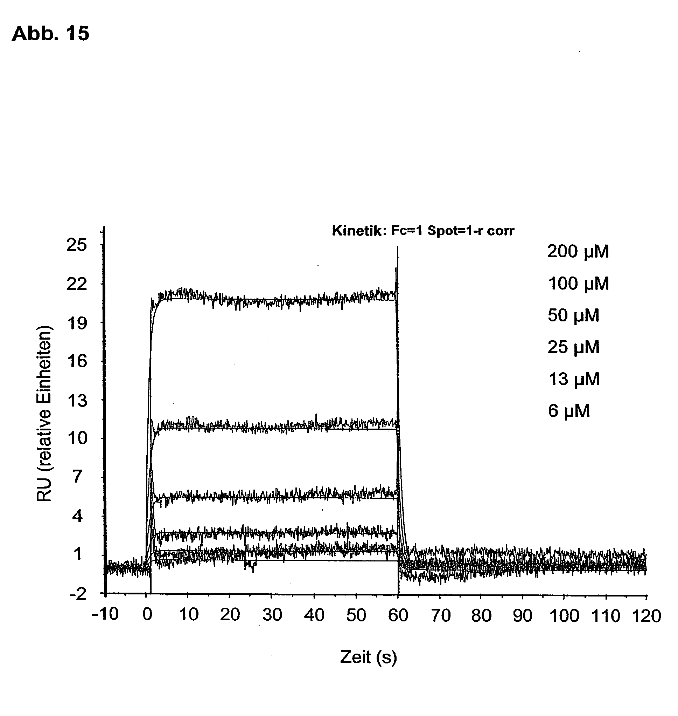

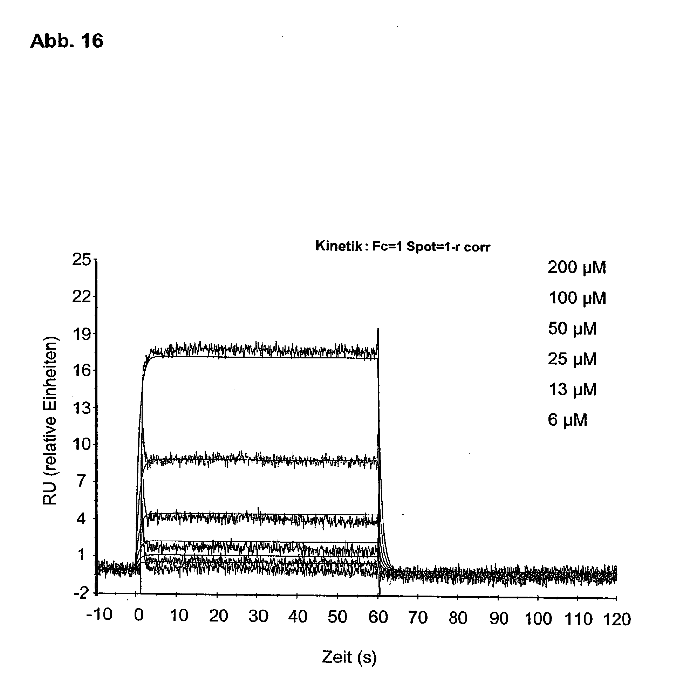

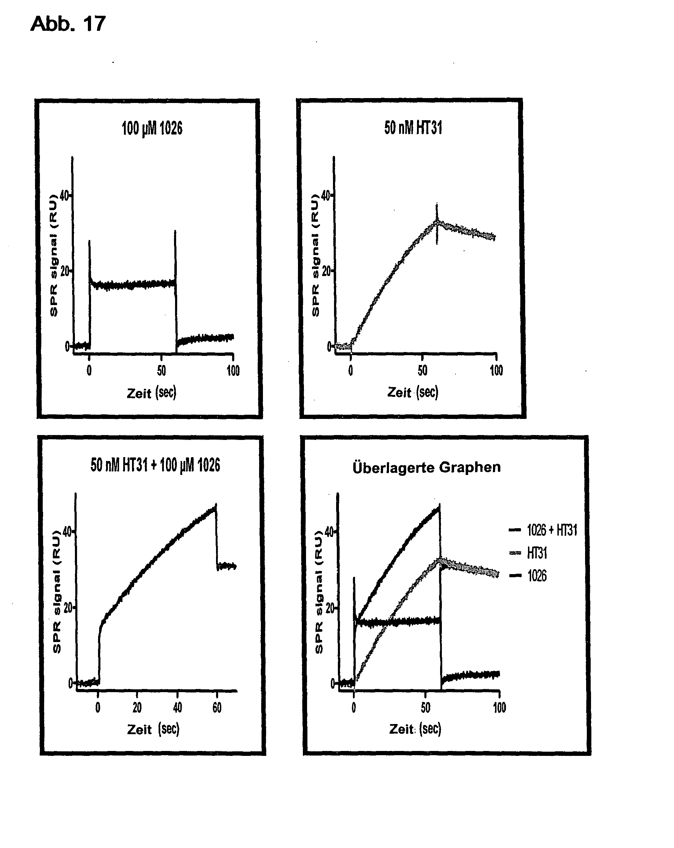

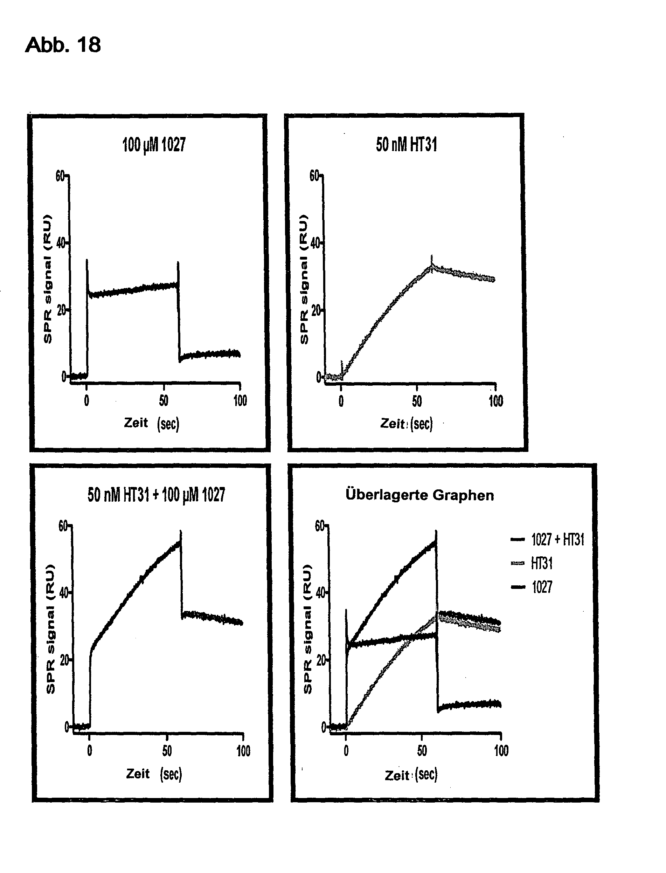

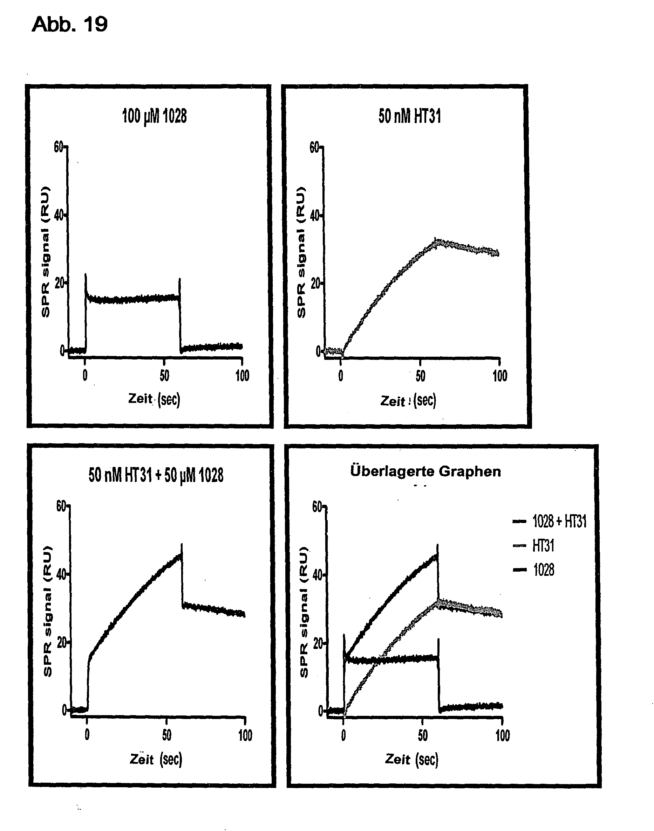

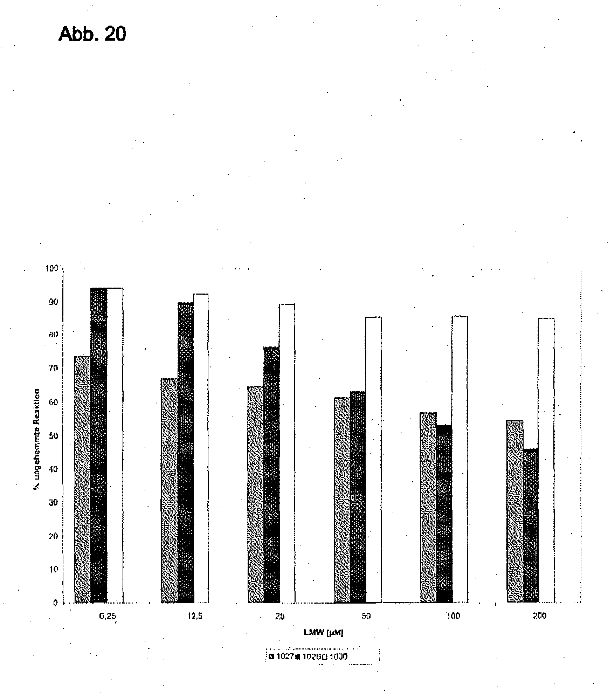

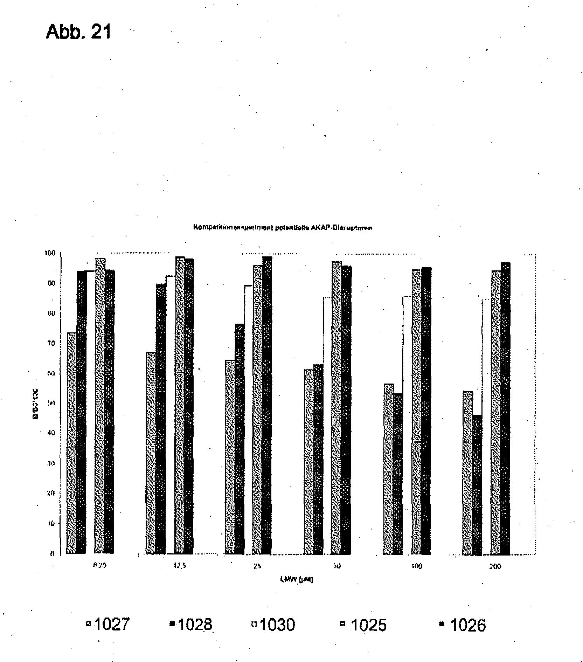

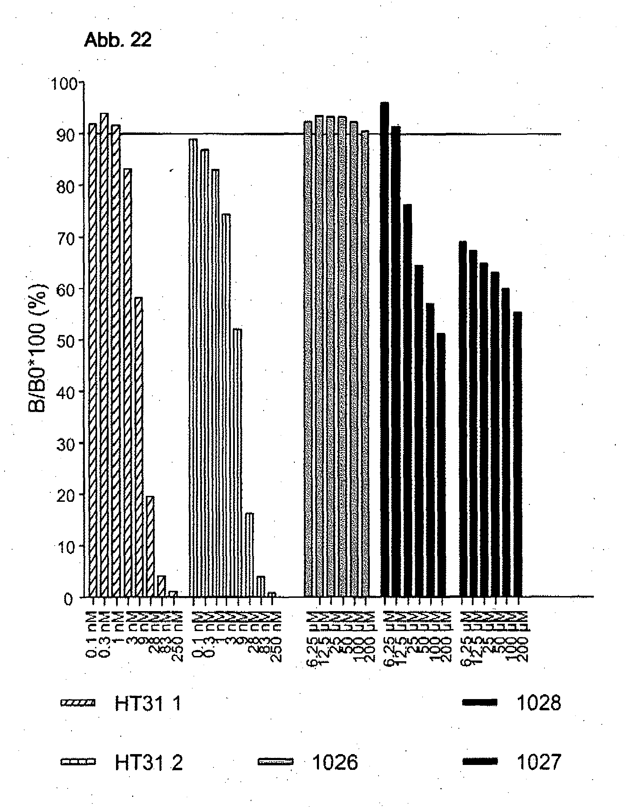

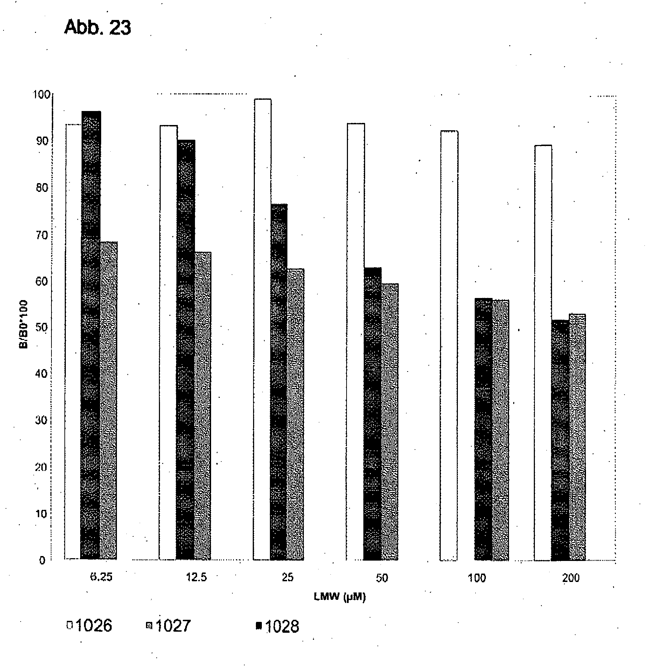

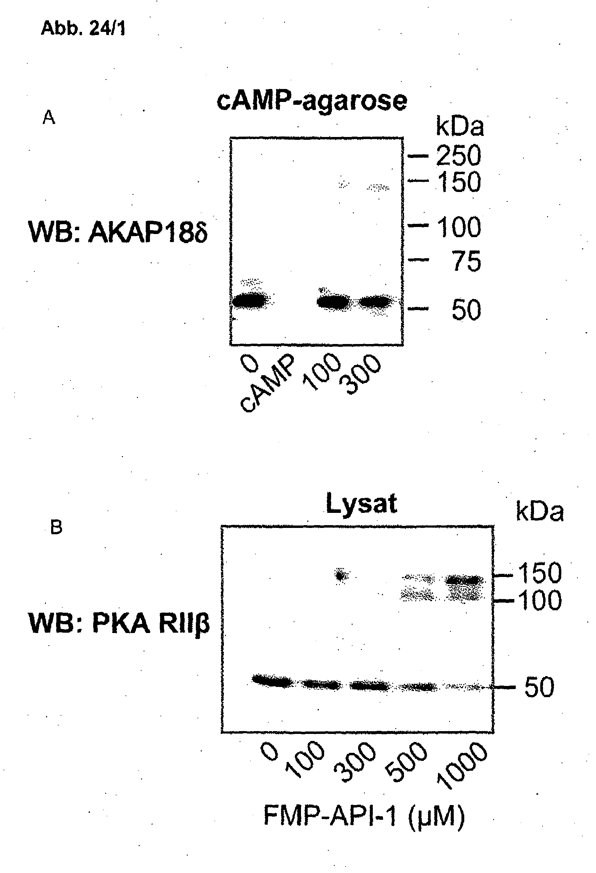

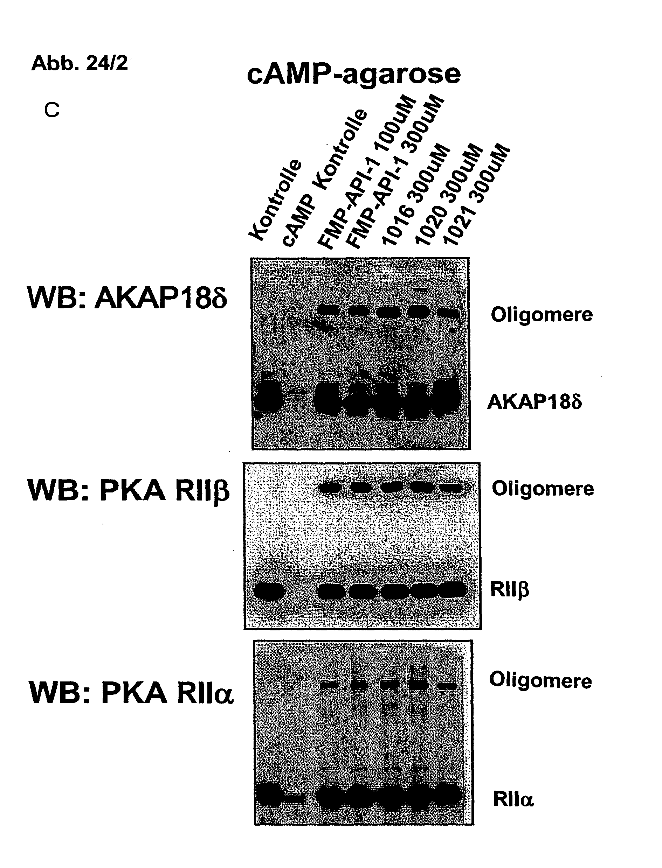

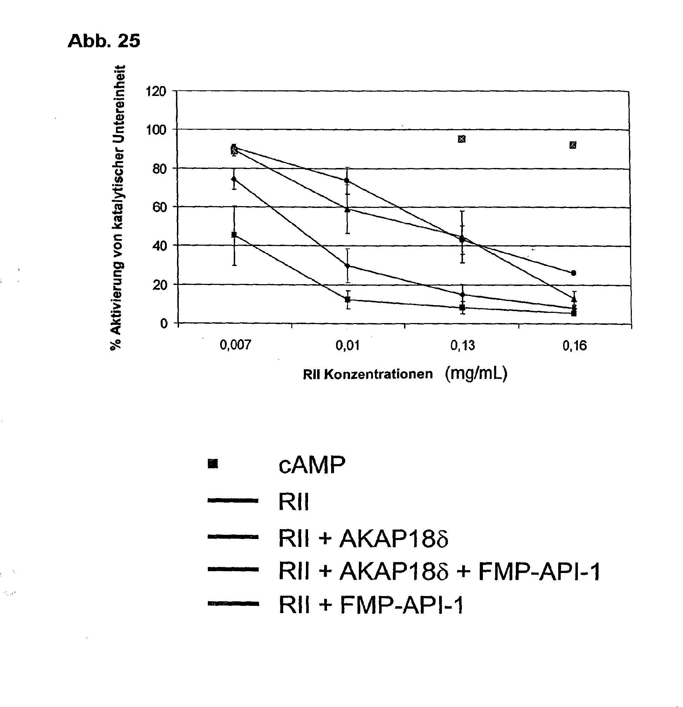

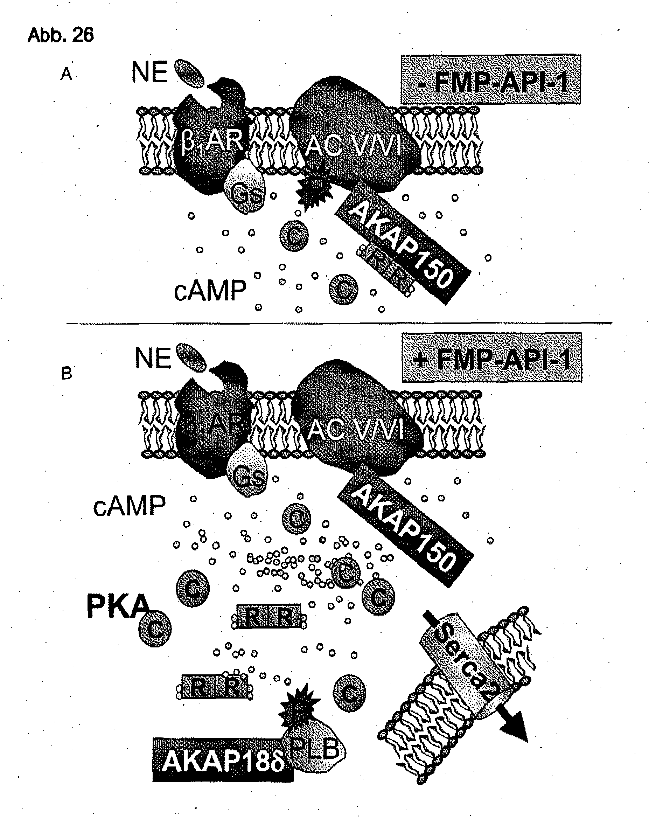

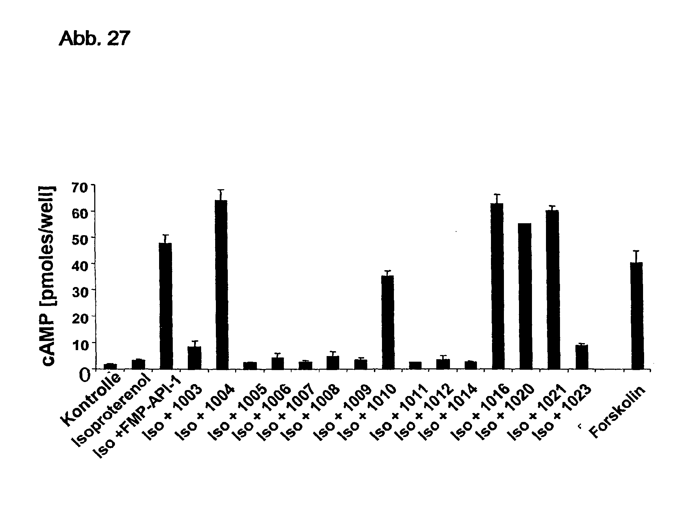

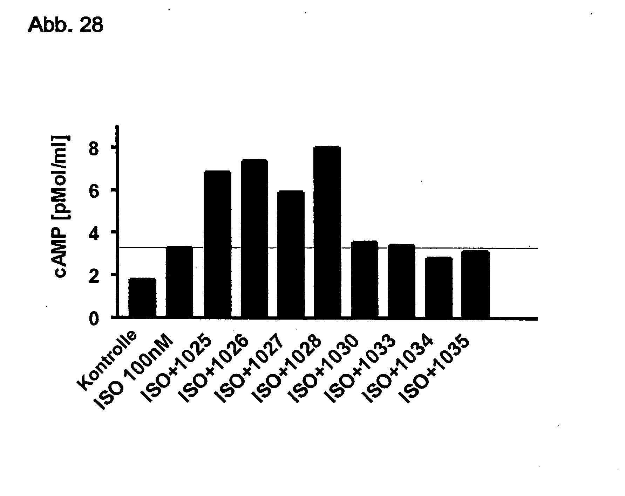

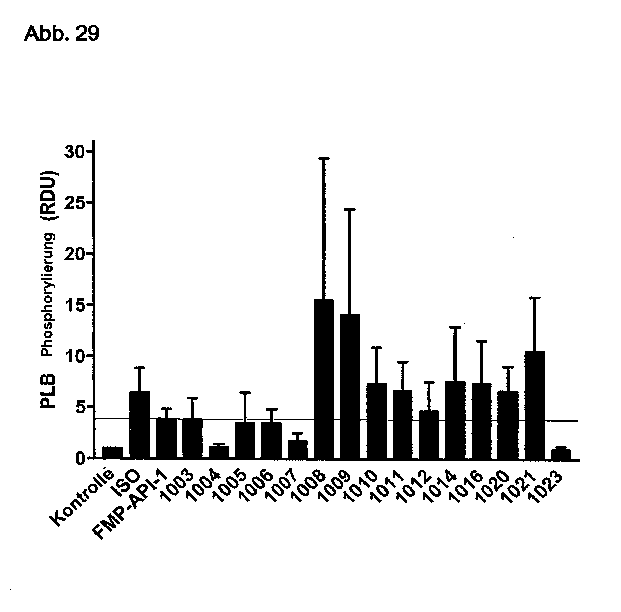

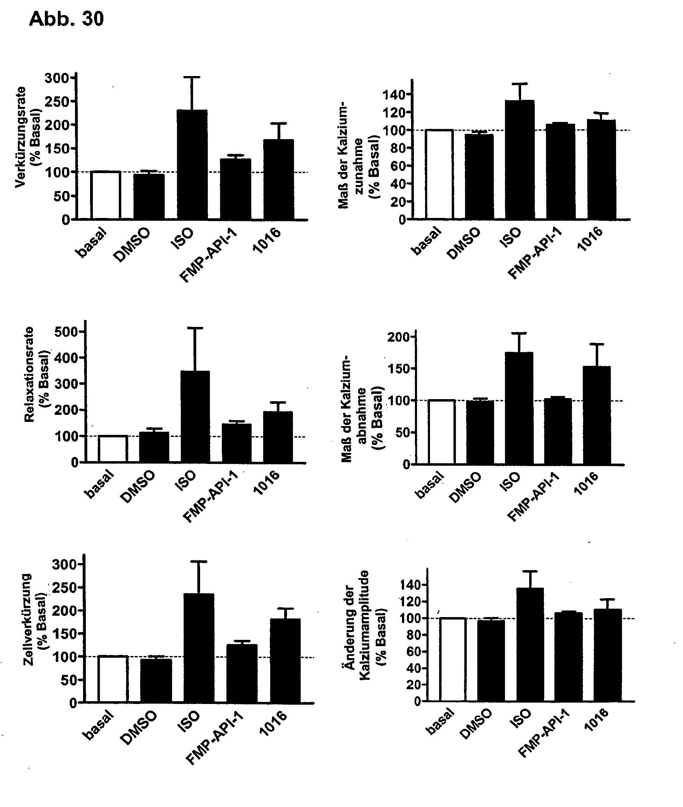

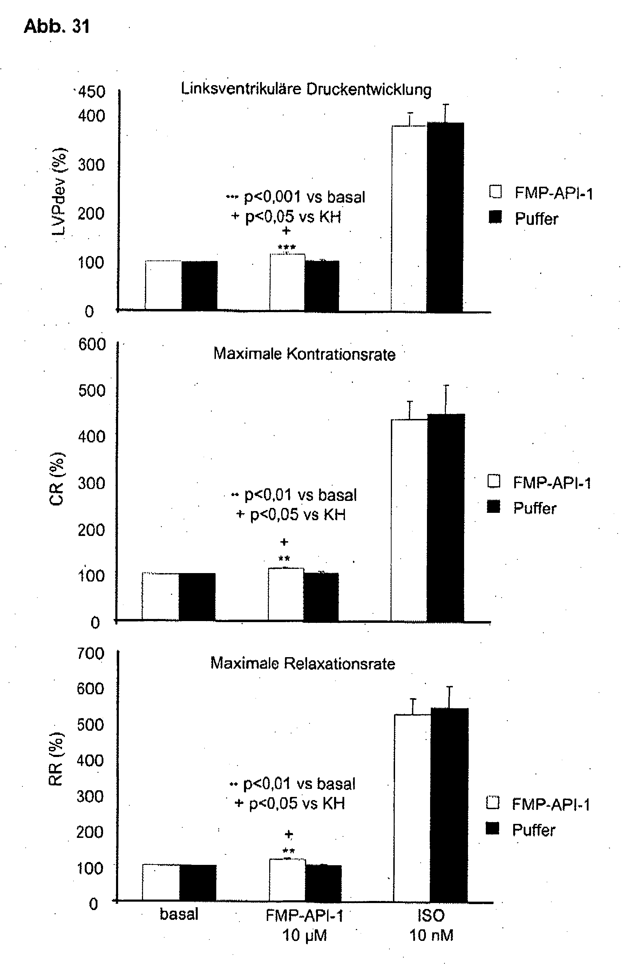

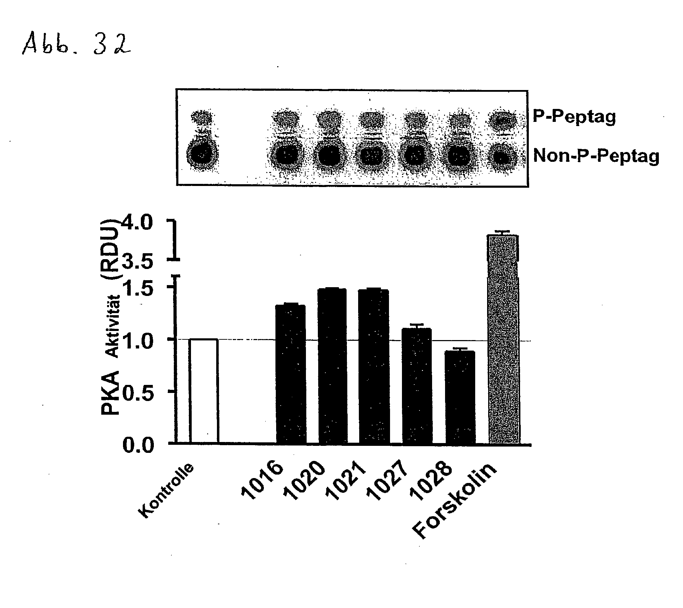

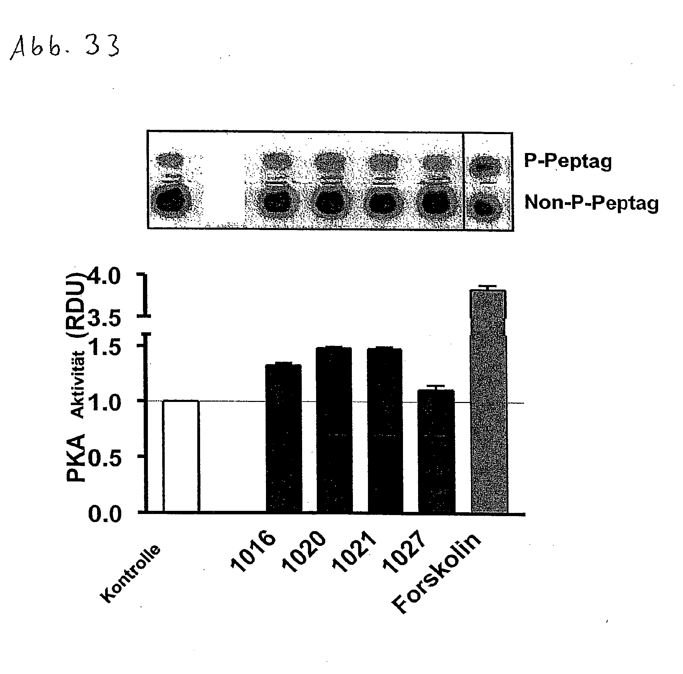

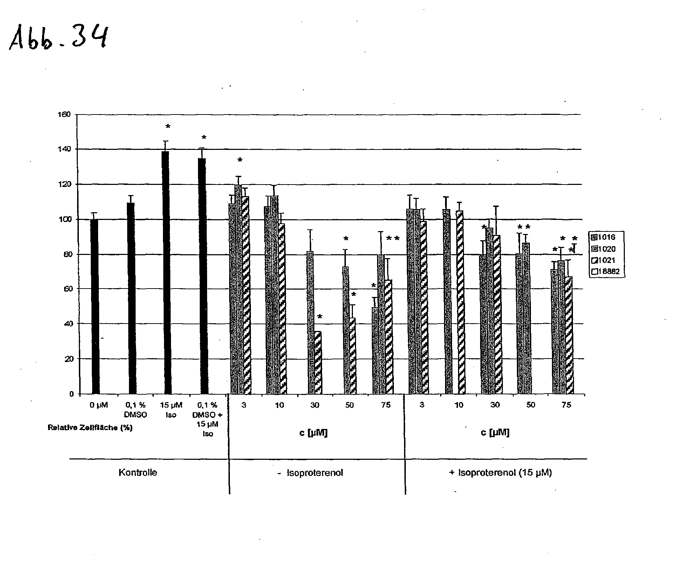

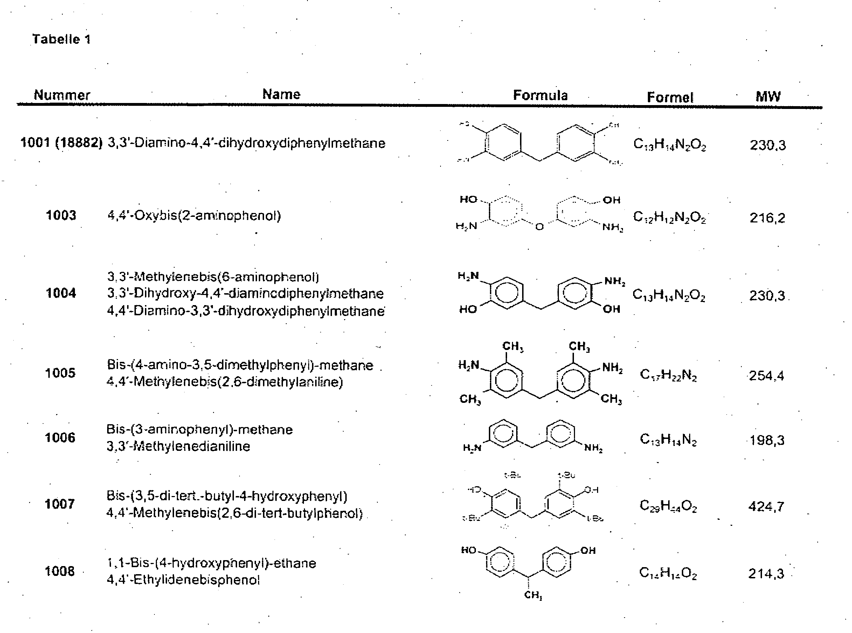

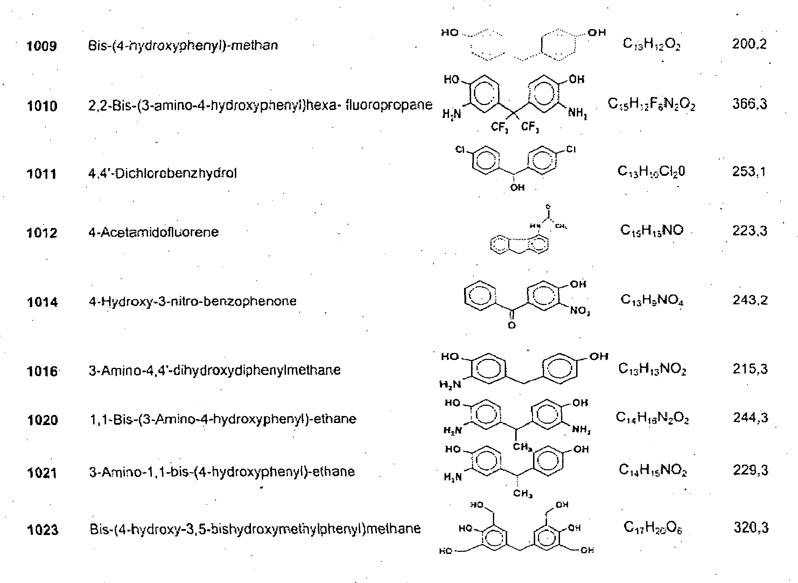

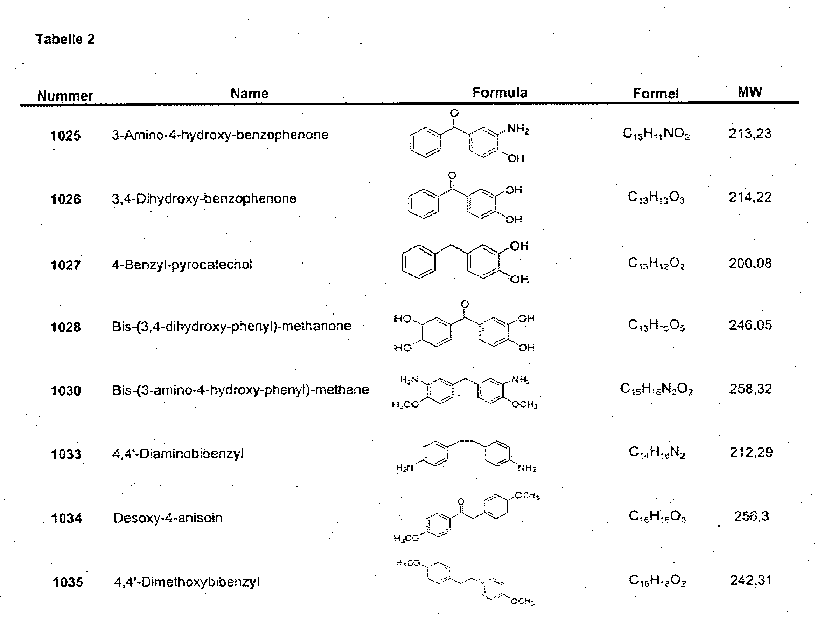

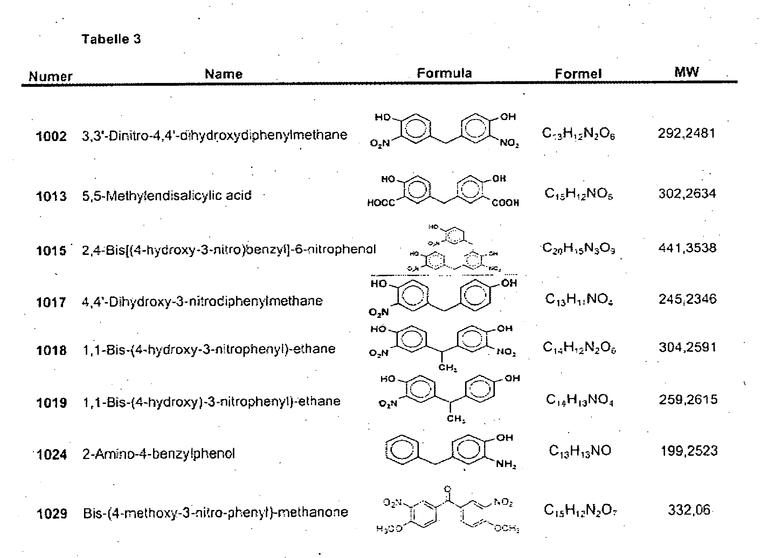

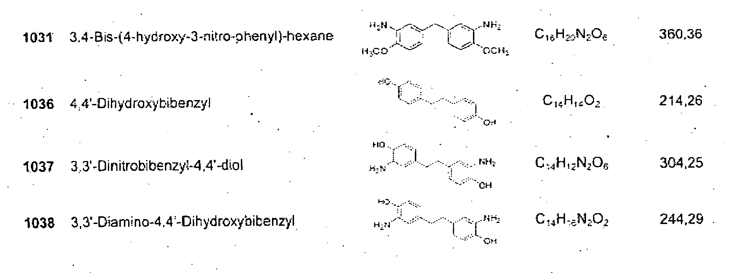

Die Substanz 18882 (FMP-API-1) aktiviert PKA in vitro. Das Experiment wurde wie in Abb. 24 dargestellt durchgeführt. Hier wurde zusätzlich mit AKAP18δ inkubiert. Katalytische Untereinheiten der PKA wurden in An- oder Abwesenheit von regulatorischen RIIβ-Untereinheiten mit der Substanz 18882 (FMP-API-1; 1 mM) inkubiert. Die Aktivierung der PKA wurde anhand der Phosphorylierung des fluoreszierenden Substratpeptids Peptag verfolgt und quantifiziert. Die semiquantitative Analyse der Peptag-Peptidphosphorylierung wurde densitometrisch vorgenommen (Mittelwerte ± SEM). Die Gegenwart des AKAP hat einen geringen zusätzlich Einfluß auf den Effekt der Substanz 18882.

### Abb. 26

AKAP150 verankert die PKA an den Adenylyzyklasen (AC) V/VI und ermöglicht dadurch die PKA-Phosphorylierung von ACV und VI nach Anstieg von cAMP, z. B. nach Aktivierung von β-Adrenozeptoren. Durch die Phosphorylierung werden diese AC gehemmt. Die Entkopplung der PKA von AKAP150 durch die Substanz 18882 (FMP-API-1) verhindert die Phosphorylierung und dadurch das Abschalten von ACV/VI nach Anstieg von cAMP. Infolge dessen kann der cAMP-Spiegel ansteigen und PKA im aktivierten Zustand erhalten, so dass diese ihre Substrate phosphoryliert.

### Abb. 27

Die Substanzen 18882 (FMP-API-1), 1004, 1010, 1016, 1020, 1021 und 1023 steigern in Gegenwart des β-Adrenozeptoragonisten Isoproterenol den cAMP-Spiegel in neonatalen Kardiomyozyten aus der Ratte. Die Zellen wurden unbehandelt belassen, mit Isoproterenol allein (100 nM), mit Isoproterenol (100 nM) und den Substanzen (50 µM) oder mit Forskolin inkubiert. Der cAMP-Spiegel wurde durch Radioimmuno-Assay (RIA) bestimmt.

### Abb. 28

Die Substanzen 1025, 1026, 1027 und 1028 steigern in Gegenwart des β-Adrenozeptoragonisten Isoproterenol den cAMP-Spiegel in neonatalen Kardiomyozyten aus der Ratte. Die Zellen wurden unbehandelt belassen, mit Isoproterenol allein (100 nM) oder mit Isoproterenol (100 nM) und den Substanzen (50 µM) inkubiert. Der cAMP-Spiegel wurde durch einen ELISA-basierten Assay bestimmt.

### Abb. 29

Die Verbindungen 18882 (FMP-API-1), 1003, 1005, 1006, 1007, 1008, 1009, 1010, 1011, 1012, 1014, 1016, 1020 und 1021 induzieren eine Zunahme der Phosphorylierung von Phopholamban (PLB) in neonatalen Kardiomyozyten. Die Zellen wurden mit DMSO (control), dem β-Adrenozeptoragonisten Isoproterenol (ISO) oder dem AKAP-PKA-Interaktionshemmer FMP-API-1 bzw. dessen Derivaten (300 µM) für 5 min. inkubiert, anschließend lysiert und im Western Blot wurde phosphoryliertes Phospholamban (p-PLB) mittels eines spezifischen anti-Phospholambanantikörpers detektiert. Calsequestrin, ein Protein des sarkoplasmatischen Retikulums, dient als Ladungskontrolle. Für die Quantifizierung (densitometrische Auswertung) wurde die p-PLB-Menge auf die Menge von Calsequestrin bezogen. Angegeben sind relative densitometrische Einheiten (RDU; Mittelwerte ± SEM, drei unabhängige Experimente).

### Abb. 30

Die Substanzen 18882 (FMP-API-1) und 1016 steigern die Kontraktilität an kultivierten adulten Kardiomyozyten (positiv inotroper Effekt). Die Zellen wurden isoliert, mit Fura-2 beladen und einer Feldstimulation ausgesetzt. Bestimmt wurden die Calciumfluxe und Zellverkürzungsparameter (cell shortening parameters) unter basalen Bedingungen und in Gegenwart von DMSO, Isoproterenol , FMP-API-1 (10µM) oder 1016 (10µM). Dargestellt sind Mittelwerte ± SEM.

### Abb. 31

Bestimmung der linksventrikulären Druckentwicklung, der maximalen Kontraktions- (CR) und Relaxationsraten (RR) von Rattenherzen (in %) in einer Langendorff-Herz-Apparatur.

### Abb. 32

Die zeigt, dass die Substanzen 1016,1020,1021 und 1027 PKA in neonatalen Kardiomyozyten aus der Ratte aktivieren. Die Zellen wurden mit DMSO (Kontrolle), die Substanzen in 300µM Konzentrationen, oder dem Adenylylzyklaseaktivator Forskolin für 30 min inkubiert. Die Aktivierung der PKA wurde anhand der Phosphorylierung des fluoreszierenden Substratpeptids Peptag verfolgt und quantifiziert. Die semiquantitative Analyse der Peptag-Peptidephosphorylierung wurde densitometrisch vorgenommen. Angegeben sind relative densitometrische Einheiten (RDU; Mittelwerte ± SD).

### Abb. 33

zeigt, dass die Substanzen 1016,1020,1021 und 1027 PKA in neonatalen Kardiomyozyten aus der Ratte aktivieren. Die Zellen wurden mit DMSO (Kontrolle), die Substanzen in 300µM Konzentrationen, oder dem Adenylylzyklaseaktivator Forskolin (FSK) für 30 min inkubiert. Die Aktivierung der PKA wurde anhand der Phosphorylierung des fluoreszierenden Substratpeptids Peptag verfolgt und quantifiziert. Die semiquantitative Analyse der Peptag-Peptidephosphorylierung wurde densitometrisch vorgenommen. Angegeben sind relative densitometrische Einheiten (RDU; Mittelwerte ± SD).

### Abb. 34

zeigt den Einfluss von Isoproterenol, DMSO, den Substanzen 1016, 1020 und 1021 auf die relative Zellgröße der Kardiomyozyten bezogen auf die durchschnittliche Zellgröße von Kontrollzellen (%). Kardiomyozyten wurden für 48h entweder unbehandelt belassen oder mit 15 µM Isoproterenol als Kontrollen inkubiert. Da die Kardiomyozyten zusätzlich zu der Substanz mit höchstens 0,1% DMSO inkubiert wurden, wurden Kardiomyozyten nur mit 0,1% für 48h inkubiert. Des Weiteren wurde ein möglicher Effekt von DMSO auf die Isoproterenolinduzierte Hypertrophie untersucht und die Zellen demnach mit 0,1% DMSO für 20 min präinkubiert und anschließend mit 15 µM Isoproterenol für 48 h inkubiert. Außerdem wurden Kardiomyozyten mit den Substanzen 1016, 1020 und 1021 in den Konzentrationen 3, 10, 30, 50 oder 75 µM für 48 h ohne Isoproterenol inkubiert. Des Weiteren wurden Kardiomyozyten mit den Substanzen 1016, 1020 und 1021 in den Konzentrationen 3, 10, 30, 50 oder 75 µM präinkubiert. Dann wurde Isoproterenol (15 µM) zu den Endkonzentrationen von 3, 10, 30, 50 oder 75 µM zugegeben und die Kardiomyozyten damit für 48 h inkubiert. Nach der Inkubation mit den Substanzen wurde α-Aktinin immunfluoreszenzmikroskopisch dargestellt. Als primärer Antikörper wurde ein anti-α-Aktinin-Antikörper (aus Maus) und als sekundärer Antikörper ein Cy3-konjugierter Ziege-anti-Maus-Antikörper verwendet. Die Flächen der Zellen wurden mit dem Werkzeug ROI des Programms "Zeiss LSM *Image Browser*" vermessen (siehe Material und Methoden) und in Excel mittels T-Test nach Student statistisch ausgewertet. (**p* ≤ 0,05 bezogen auf die unbehandelte Kontrolle). Herzen wurden mit Krebs-Henseleit-Puffer, mit dem AKAP-PKA-Interaktionshemmer 18882 (FMP-API-1; 10 µM), dem b-Adrenozeptoragonisten Isoproterenol (ISO) oder den angegebenen Kombinationen perfundiert. FMP-API-1 induziert bei den gemessenen Parametern eine signifikante Zunahme um etwa 17 % (n ≥ 8 Herzen für jede experimentelle Bedingung).

## Patentansprüche

1. Verwendung einer Verbindung der Formel (I) 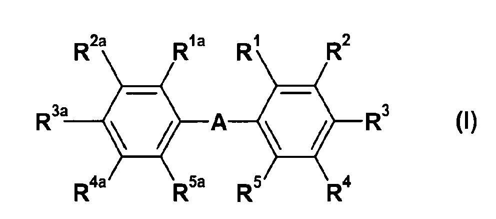 wobei
A ein Sauerstoffatom, ein Schwefelatom, eine NH, CO, Alkylen, Alkenylen, oder Heteroalkylengruppe ist,
R¹ ein Wasserstoffatom, ein Halogenatom, eine NH₂, OH, NO₂, Alkyl, Alkenyl, Alkinyl oder Heteroalkylgruppe ist,
R^{1a} ein Wasserstoffatom, ein Halogenatom, eine NH₂, OH, NO₂, Alkyl, Alkenyl, Alkinyl oder Heteroalkylgruppe ist,
R² ein Wasserstoffatom, ein Halogenatom, eine NH₂, OH, NO₂, Alkyl, Alkenyl, Alkinyl oder Heteroalkylgruppe ist,
R^{2a} ein Wasserstoffatom, ein Halogenatom, eine NH₂, OH, NO₂, Alkyl, Alkenyl, Alkinyl oder Heteroalkylgruppe ist,
R³ ein Wasserstoffatom, ein Halogenatom, eine NH₂, OH, NO₂, Alkyl, Alkenyl, Alkinyl oder Heteroalkylgruppe ist,
R^{3a} ein Wasserstoffatom, ein Halogenatom, eine NH₂, OH, NO₂, Alkyl, Alkenyl, Alkinyl oder Heteroalkylgruppe ist,
R⁴ ein Wasserstoffatom, ein Halogenatom, eine NH₂, OH, NO₂, Alkyl, Alkenyl, Alkinyl oder Heteroalkylgruppe ist,
R^{4a} ein Wasserstoffatom, ein Halogenatom, eine NH₂, OH, NO₂, Alkyl, Alkenyl, Alkinyl oder Heteroalkylgruppe ist,
R⁵ ein Wasserstoffatom, ein Halogenatom, eine NH₂, OH, NO₂, Alkyl, Alkenyl, Alkinyl oder Heteroalkylgruppe ist,
R^{5a} ein Wasserstoffatom, ein Halogenatom, eine NH₂, OH, NO₂, Alkyl, Alkenyl, Alkinyl oder Heteroalkylgruppe ist, oder
die Reste R¹ und R^{1a} oder die Reste R⁵ und R^{5a} zusammen eine Bindung sind,
oder eines pharmakologisch akzeptablen Salzes, Solvats, Hydrats oder einer pharmakologisch akzeptablen Formulierung derselben,
zur Herstellung eines Medikamentes für die Prophylaxe oder Behandlung von Krankheiten, die mit Defekten einer kompartimentalisierten cAMP-abhängigen Signaltransduktion assoziiert sind.

2. Verwendung einer Verbindung nach Anspruch 1, wobei A ein Sauerstoffatom, ein Schwefelatom, eine NH, CO, Alkylen, Alkenylen, oder Heteroalkylengruppe ist,
R¹ ein Wasserstoffatom, ein Halogenatom, eine NH₂, OH, NO₂, Alkyl, Alkenyl, Alkinyl oder Heteroalkylgruppe ist,
R^{1a} ein Wasserstoffatom, ein Halogenatom, eine NH₂, OH, NO₂, Alkyl, Alkenyl, Alkinyl oder Heteroalkylgruppe ist,
R² ein Wasserstoffatom, ein Halogenatom, eine NH₂, OH, NO₂, Alkyl, Alkenyl, Alkinyl oder Heteroalkylgruppe ist,
R^{2a} ein Wasserstoffatom, ein Halogenatom, eine NH₂, OH, NO₂, Alkyl, Alkenyl, Alkinyl oder Heteroalkylgruppe ist,
R³ ein Wasserstoffatom, ein Halogenatom, eine NH₂, OH, NO₂, Alkyl, Alkenyl, Alkinyl oder Heteroalkylgruppe ist,
R^{3a} ein Wasserstoffatom, ein Halogenatom, eine NH², OH, NO₂, Alkyl, Alkenyl, Alkinyl oder Heteroalkylgruppe ist,
R⁴ ein Wasserstoffatom, ein Halogenatom, eine NH₂, OH, NO₂, Alkyl, Alkenyl, Alkinyl oder Heteroalkylgruppe ist,
R^{4a} ein Wasserstoffatom, ein Halogenatom, eine NH₂, OH, NO₂, Alkyl, Alkenyl, Alkinyl oder Heteroalkylgruppe ist,
R⁵ ein Wasserstoffatom, ein Halogenatom, eine NH₂, OH, NO₂, Alkyl, Alkenyl, Alkinyl oder Heteroalkylgruppe ist, und
R^{5a} ein Wasserstoffatom, ein Halogenatom, eine NH₂, OH, NO₂, Alkyl, Alkenyl, Alkinyl oder Heteroalkylgruppe ist.

3. Verwendung einer Verbindung nach Anspruch 1 oder 2, wobei die Reste R¹, R^{1a}, R², R^{2a}, R³, R^{3a}, R⁴, R^{4a}, R⁵ und R^{5a} unabhängig voneinander H, F, Cl, NH₂, OH, NO₂, C₁-C₄ Alkylgruppen oder C₁-C₄ Heteroalkylgruppen sind.

4. Verwendung einer Verbindung nach einem der voranstehenden Ansprüche, wobei die kürzeste Kette der Gruppe A zwischen den beiden Phenylringen eine Länge von ein oder zwei Atomen, ausgewählt aus C oder O hat.

5. Verwendung einer Verbindung nach einem der voranstehenden Ansprüche, wobei R¹ ein Wasserstoffatom ist, R^{1a} ein Wasserstoffatom ist, R² ein Wasserstoffatom oder eine Gruppe der Formel NH₂, OH, CH₃, ^{t}Bu, NHCOCH₃, NO₂ oder CH₂OH ist, R^{2a} ein Wasserstoffatom oder eine Gruppe der Formel NH₂, OH, CH₃, ^{t}Bu, NHCOCH₃, NO₂ oder CH₂OH ist, R³ ein Wasserstoffatom, ein Chloratom oder eine Gruppe der Formel NH₂, OH oder OCH₃ ist, R^{3a} ein Wasserstoffatom, ein Chloratom oder eine Gruppe der Formel NH₂, OH oder OCH₃ ist, R⁴ ein Wasserstoffatom oder eine Gruppe der Formel NH₂, OH, CH₃, ^{t}Bu, NHCOCH₃, NO₂ oder CH₂OH ist, R^{4a} ein Wasserstoffatom oder eine Gruppe der Formel NH₂, OH, CH₃, ^{t}Bu, NHCOCH₃, NO₂ oder CH₂OH ist, R⁵ ein Wasserstoffatom ist und R^{5a} ein Wasserstoffatom ist.

6. Verwendung einer Verbindung nach einem der voranstehenden Ansprüche, wobei von den Resten R², R^{2a}, R⁴ und R^{4a} maximal einer eine NH₂ Gruppe ist oder mindestens drei eine NH₂ Gruppe sind, wenn R³ und R^{3a} eine OH Gruppe sind.

7. Verwendung einer Verbindung nach einem der voranstehenden Ansprüche, wobei R³ eine OH-Gruppe ist und R² und R⁴ keine NH₂ Gruppen sind.

8. Verwendung einer Verbindung nach einem der voranstehenden Ansprüche, wobei A aus den folgenden Gruppen ausgewählt ist: O, S, NH, NCH₃, CH₂, CH(CH₃), C(CF₃)₂, CH(OH), CO, CH₂CH₂, oder COCH₂.

9. Verwendung einer Verbindung der Formel (X), 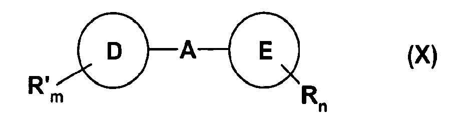 wobei
A ein Sauerstoffatom, ein Schwefelatom, eine NH, CO, Alkylen, Alkenylen, Alkinylen, Heteroalkylen, Arylen, Heteroarylen, Cycloalkylen, Alkylcycloalkylen, Heteroalkylcycloalkylen, Heterocycloalkylen, Aralkylen oder eine Heteroaralkylengruppe ist,
die Reste R unabhängig voneinander ein Wasserstoffatom, ein Halogenatom, ein NH₂, OH, SH, N₃, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
die Reste R' unabhängig voneinander ein Wasserstoffatom, ein Halogenatom, ein NH₂, OH, SH, N₃, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
D ein Aryl-, Heteroaryl-, Cycloalkyl-, Alkylcycloalkyl-, Heteroalkylcycloalkyl-, Heterocycloalkyl-, Aralkyl- oder ein Heteroaralkylring ist,
E ein Aryl-, Heteroaryl-, Cycloalkyl-, Alkylcycloalkyl-, Heteroalkylcycloalkyl-, Heterocycloalkyl-, Aralkyl- oder ein Heteroaralkylring ist,
n gleich 0, 1, 2, 3, 4 oder 5 ist und
m gleich 0, 1, 2, 3, 4 oder 5 ist,
oder eines pharmakologisch akzeptablen Salzes, Solvats, Hydrats oder einer pharmakologisch akzeptablen Formulierung derselben,
zur Herstellung eines Medikamentes für die Prophylaxe oder Behandlung von Krankheiten, die mit Defekten einer kompartimentalisierten cAMP-abhängigen Signaltransduktion assoziiert sind.

10. Verwendung nach Anspruch 9, wobei A ein Sauerstoffatom, ein Schwefelatom, eine NH, CO, Alkylen, Alkenylen, oder Heteroalkylengruppe ist, D eine Phenylgruppe oder eine Heteroarylgruppe mit 5 oder 6 Ringatomen ist, E eine Phenylgruppe oder eine Heteroarylgruppe mit 5 oder 6 Ringatomen ist, die Reste R unabhängig voneinander ein ein Wasserstoffatom, ein Halogenatom, eine NH₂, OH, NO₂, Alkyl, Alkenyl, Alkinyl oder Heteroalkylgruppe sind,
die Reste R' unabhängig voneinander ein Wasserstoffatom, ein Halogenatom, eine NH₂, OH, NO₂, Alkyl, Alkenyl, Alkinyl oder Heteroalkylgruppe sind, n 1, 2 oder 3 ist und m 1, 2 oder 3 ist.

11. Verwendung einer Verbindung der Formel (I), 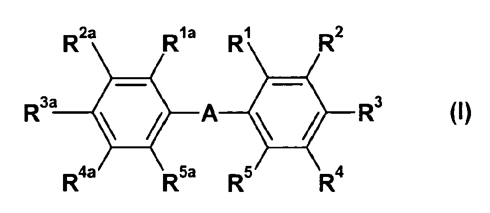 wobei
A ein Sauerstoffatom, ein Schwefelatom, eine NH, CO, Alkylen, Alkenylen, Alkinylen, Heteroalkylen, Arylen, Heteroarylen, Cycloalkylen, Alkylcycloalkylen, Heteroalkylcycloalkylen, Heterocycloalkylen, Aralkylen oder eine Heteroaralkylengruppe ist und
die Reste R¹, R^{1a}, R², R^{2a}, R³, R^{3a}, R⁴, R^{4a}, R⁵ und R^{5a} unabhängig voneinander ein Wasserstoffatom, ein Halogenatom, ein NH₂, OH, SH, N₃, NO₂, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest sind,
oder eines pharmakologisch akzeptablen Salzes, Solvats, Hydrats oder einer pharmakologisch akzeptablen Formulierung derselben,
zur Herstellung eines Medikamentes für die Prophylaxe oder Behandlung von Krankheiten, die mit Defekten einer kompartimentalisierten cAMP-abhängigen Signaltransduktion assoziiert sind.

12. Verwendung nach einem der voranstehenden Ansprüche, wobei die Krankheiten ausgewählt sind aus: Hypertrophie des Herzens, einer koronaren Herzkrankheit, Hypertonie und Herzinsuffizienz.
